# EUROPÄISCHE PATENTANMELDUNG

(11) **EP 4 617 731 A1**
(43) Veröffentlichungstag der Anmeldung: **17.09.2025**
(21) Anmeldenummer: 24163525.9
(22) Anmeldetag: 14.03.2024
(51) Int. Cl.: G01T 1/172, G01T 1/24, G01T 1/29

(54) **ABSCHÄTZEN EINER RATE VON ZUFÄLLIGEN KOINZIDENZEN IN EINEM ZÄHLENDEN RÖNTGENDETEKTOR**

(71) Anmelder: Siemens Healthineers AG, 91301 Forchheim (DE)
(72) Erfinder: Göderer, Edgar, 91301 Forchheim (DE); Hosemann, Michael, 91056 Erlangen (DE); Hupfer, Martin, 91052 Erlangen (DE); Konrad, Sebastian, 91301 Forchheim (DE); Kreisler, Björn, 91353 Hausen (DE); Reitz, Bodo, 91301 Forchheim (DE)
(74) Vertreter: Siemens Healthineers Patent Attorneys

(57) **Zusammenfassung**

Verfahren zum Abschätzen einer Rate von zufälligen Koinzidenzen in einem zählenden Röntgendetektor (31) mit eine Vielzahl von Detektorelementen, wobei das Verfahren die folgenden Schritte umfasst:
- Erfassen von Röntgensignalen durch den Röntgendetektor (31) und Umwandeln der Röntgensignale in elektrische Signale;
- Weiterleiten von zumindest einigen der elektrischen Signale zu Signaleingängen einer Koinzidenzeinheit (2), wobei die Signaleingänge einen ersten Signaleingang und zumindest einen weiteren Signaleingang umfassen,
- wobei die Signale für den ersten Signaleingang in einem ersten der Detektorelemente erfasst werden,
- wobei die Signale für den zumindest einen weiteren Signaleingang jeweils in einem anderen nicht direkt zu dem ersten der Detektorelemente benachbarten Detektorelement erfasst werden
und/oder
wobei Signale für den zumindest einen weiteren Signaleingang oder die Signale für den ersten Signaleingang in einer elektrischen Schaltung mit einem definierten Zeitintervall zeitlich versetzt werden;
- Zählen von Koinzidenzen der in die Koinzidenzeinheit (2) weitergeleiteten Signale und Abschätzen einer Rate von zufälligen Koinzidenzen.

## Beschreibung

Die Erfindung betrifft ein Verfahren zum Abschätzen einer Rate von zufälligen Koinzidenzen in einem zählenden Röntgendetektor, ein Verfahren zum Abschätzen einer echten Koinzidenz, ein Verfahren zum Aufnehmen eines Röntgenbilddatensatzes, einen zählenden Röntgendetektor und ein medizinisches Bildgebungsgerät.

Unabhängig vom grammatikalischen Geschlecht eines bestimmten Begriffs sind Personen mit männlicher, weiblicher oder anderer Geschlechteridentität mit umfasst.

Photonenzählende Röntgendetektoren werden für viele verschiedene Anwendungen, insbesondere für bildgebende Anwendungen, eingesetzt. Beispielsweise werden photonenzählende Detektoren zunehmend für den Einsatz in Computertomographiesystemen (CT-Systemen) verwendet. Photonenzählende Röntgendetektoren basieren in der Regel darauf, dass eintreffende Röntgensignale bzw. Röntgenphotonen durch einen Konverter in elektrische Signale umgewandelt werden, die dann registriert und ausgewertet werden können. Es können Röntgendetektoren mit einstellbaren Energieschwellen verwendet werden, die eine energieaufgelöste Erfassung der Röntgensignale ermöglichen.

Um einerseits eine hohe Ortsauflösung zu erreichen und andererseits die Zählraten in den einzelnen Detektorelementen zu begrenzen, werden die Detektorelemente oder Pixelelemente tendenziell sehr klein ausgelegt. Dies kann allerdings den Nachteil haben, dass häufig nicht die gesamte Energie eines Röntgenquants in einem Pixel deponiert wird, sondern sich über zwei oder mehr - typischerweise benachbarte - Detektorelemente bzw. Pixel verteilt, da die im Detektor erzeugten Ladungswolken sich über mehr als ein Detektorelement ausdehnen. Dadurch kann es vorkommen, dass Photonen in benachbarten Detektorelementen mehrfach gezählt werden. Dadurch kann sowohl die Ortsauflösung als auch die Energieauflösung des Detektorsystems begrenzt werden. Das Auftreten dieser Doppelungen kann als Koinzidenzen, bzw. wahre Koinzidenzen oder echte Koinzidenzen bezeichnet werden.

Ein Ansatz zur Lösung dieses Problems ist es, für jedes Detektorelement zusätzlich zu einem Zähler für die Zählraten der erfassten Photonen noch einen Koinzidenzzähler einzuführen, der Ereignisse zählt, bei denen mindestens ein benachbartes Detektorelement zeitgleich mit dem betrachteten Detektorelement registriert wird. Die Zählraten der Zähler und Koinzidenzzähler können insbesondere für vorhandene Energieschwellen erfasst werden, sodass ein Koinzidenzzähler Ereignisse zählt, bei denen mindestens ein Nachbarpixel zeitgleich mit dem betrachteten Pixel eine Energieschwelle überschreitet.

Ansätze zum Erfassen der Koinzidenzen sind im Stand der Technik bekannt und werden beispielsweise in DE 10 2012 224 209 A1, EP 3 839 577 A1 und EP 3 839 576 A1 beschrieben.

Solche Koinzidenzzähler haben allerdings oftmals die Tendenz, die Häufigkeit der Röntgenquanten, deren Signal auf mehrere Pixel aufgeteilt ist, zu überschätzen. Dies liegt daran, dass der Koinzidenzzähler nicht nur im Falle von echten Koinzidenzen erhöht wird, sondern auch dann, wenn zeitgleich mit dem im betrachteten Pixel eintreffenden Röntgenquant zufällig ein zweites, unabhängiges Röntgenquant in einem der Nachbarpixel seine Energie deponiert - auch als zufällige Koinzidenzen bezeichnet. Bei dem Auftreten von zufälligen Koinzidenzen kann die Verwendung des Koinzidenzzählers zu einer Überkorrektur führen. Typischerweise nimmt die Größe des Fehlers durch die Nichtbeachtung zufälliger Koinzidenzen mit höheren Photonenflüssen stark zu. Dies liegt daran, dass die Anzahl dieser Ereignisse sich in erster Näherung aus dem Produkt der Zählrate im Pixel, der Zählraten im Nachbarpixel, der Anzahl der Nachbarpixel und der Länge des Koinzidenzzeitfensters ergibt, während die echten Koinzidenzen nur einen gewissen Anteil der Zählrate im betrachteten Pixel darstellt.

Eine Abschätzung der Anzahl der zufälligen Koinzidenzen aus den Einzelzählraten aller Pixel ist nur begrenzt möglich, da hierzu der exakte Anteil der echten Koinzidenzen an den Einzelzählraten bekannt sein müsste, was in einem CT-System aber typischerweise nicht der Fall ist, da dieser Anteil wiederum spektral abhängig ist.

Es ist daher eine Aufgabe der vorliegenden Erfindung, eine Möglichkeit bereitzustellen, welche die Problematik einer überschätzten echten Koinzidenz bei zählenden Röntgendetektoren zumindest verringern bzw. eine bessere Abschätzung einer echten Koinzidenz bereitstellen kann.

Diese Aufgabe wird gelöst durch ein Verfahren gemäß Anspruch 1, ein Verfahren gemäß Anspruch 7, ein Verfahren gemäß Anspruch 13, einen zählenden Röntgendetektor gemäß Anspruch 14 und ein medizinisches Bildgebungsgerät gemäß Anspruch 15. Weitere Merkmale und Vorteile ergeben sich aus den abhängigen Ansprüchen, der Beschreibung und den beigefügten Figuren.

Gemäß einem ersten Aspekt der Erfindung ist ein Verfahren zum Abschätzen einer Rate von zufälligen Koinzidenzen in einem zählenden Röntgendetektor vorgesehen. Der Röntgendetektor umfasst eine Vielzahl von Detektorelementen. Das Verfahren umfasst die folgenden Schritte:
(a) Erfassen von Röntgensignalen durch den Röntgendetektor und Umwandeln der Röntgensignale in elektrische Signale an den Detektorelementen;
(b) Weiterleiten von zumindest einigen der elektrischen Signale zu Signaleingängen einer Koinzidenzeinheit, wobei die Signaleingänge einen ersten Signaleingang und zumindest einen weiteren Signaleingang umfassen,
   - wobei die Signale für den ersten Signaleingang in einem ersten der Detektorelemente erfasst werden,
   - wobei die Signale für den zumindest einen weiteren Signaleingang jeweils in einem anderen nicht direkt zu dem ersten der Detektorelemente benachbarten Detektorelement erfasst werden
   und/oder
   wobei Signale für den zumindest einen weiteren Signaleingang oder die Signale für den ersten Signaleingang in einer elektrischen Schaltung mit einem definierten Zeitintervall zeitlich versetzt werden, bevor sie zu der Koinzidenzeinheit weitergeleitet werden;
(c) Zählen von Koinzidenzen der in die Koinzidenzeinheit weitergeleiteten Signale, um zumindest eine Zählrate von erfassten zufälligen Koinzidenzen zu bestimmen;
(d) Abschätzen einer Rate von zufälligen Koinzidenzen anhand der zumindest einen bestimmten Zählrate.

Vorteilhafterweise stellt das erfindungsgemäße Verfahren eine Möglichkeit dar, zufällige Koinzidenzen relativ zuverlässig abzuschätzen. Dies kann es beispielsweise ermöglichen, den nutzbaren Bereich für Koinzidenzzähler zu höheren Röntgenflüssen auszudehnen, indem durch Kenntnis der zufälligen Koinzidenzen der Beiträge von echten Koinzidenzen und den zufälligen Koinzidenzen aufgeschlüsselt werden kann.

Zufällige Koinzidenzen können auch als unabhängige Koinzidenzen oder falsche Koinzidenzen oder als Zufallskoinzidenzen bezeichnet werden. Der Begriff "zufällige Koinzidenzen" ist in Abgrenzung zu dem Begriff "echte Koinzidenzen" zu verstehen. Zufällige Koinzidenzen treten auf, wenn zufällig gleichzeitig zwei primären Röntgenquanten in verschiedenen Detektorelemente, insbesondere benachbarten Detektorelementen auftreffen. Verschiedene Faktoren, wie zum Beispiel der Röntgenfluss und die Breite der Detektorelemente können die Rate der zufälligen Koinzidenzen beeinflussen. Echte Koinzidenzen können auch als wahre Koinzidenzen bezeichnet werden. Echte Koinzidenzen treten auf, wenn das gleiche Ereignis, also insbesondere ein einzelnes Röntgenquant, gleichzeitig ein Signal in benachbarten Detektorelementen auslöst. Mit zählenden Röntgendetektoren wird versucht, die korrekte Zahl an Röntgenquanten und auch deren korrekte Energie zu erfassen. Durch den Ansatz, Koinzidenzen zu erfassen, kann darauf rückgeschlossen werden, wenn einzelne Photonen bzw. Röntgenquanten mehrere Signale auslösen, sodass die korrekte Anzahl an Röntgenquanten besser bestimmt werden kann. Diese Bestimmung kann jedoch durch die zufälligen Koinzidenzen verschlechtert werden, wodurch eine Überkorrektur induziert werden kann, indem mehr echte Koinzidenzen gezählt werden als eigentlich vorhanden sind. Vorteilhafterweise können durch das erfindungsgemäße Verfahren zufällige Koinzidenzen bestimmt werden, womit folglich, gerade bei großen Röntgenflüssen, die echten Koinzidenzen präziser ermittelt bzw. entsprechende Zählraten korrigiert werden können.

Der zählende Röntgendetektor kann beispielsweise ein zählender Röntgendetektor eines Computertomographiesystems sein. Der zählende Röntgendetektor kann auch als photonenzählender Röntgendetektor bezeichnet werden. Er ist allgemein darauf ausgelegt, einzelne Röntgenphotonen, insbesondere örtlich und/oder zeitlich aufgeschlüsselt, zu erfassen und zu zählen. Der Begriff Röntgensignale ist im Rahmen dieser Erfindung breit zu verstehen. Mit Röntgensignalen können allgemein eintreffende Röntgenstrahlen bzw. Röntgenphotonen bezeichnet werden. Typischerweise umfasst ein zählender Röntgendetektor einen Röntgenkonverter, in dem einfallende Röntgenstrahlen bewegliche Ladungsträger, insbesondere Elektronen-Loch-Paare, erzeugen. An dem Röntgenkonverter sind typischerweise elektrische Kontakte als Elektroden angelegt, an denen eine Spannung angelegt wird. Durch die Spannung werden die erzeugten Ladungsträger zu Kontakten und daran anschließende Ausleseelektronik transportiert, wo sie typischerweise verstärkt, die Signalhöhen gegen Schwellen verglichen und beim Überschreiten einer definierten Schwelle in auszugebende elektrische Signale, insbesondere logische und/oder digitale elektrische Signale, gewandelt werden. Elektrische Signale können im Rahmen dieser Erfindung auch kurz als Signale bezeichnet werden. Die Kontakte zusammen mit zumindest Teilen der angeschlossenen Ausleseelektronik sind Teil der Detektorelemente. Die eingehenden Signale können von Zählern in der Ausleseelektronik gezählt werden. Bei zählenden Röntgendetektoren, insbesondere im Kontext der Computertomographie, werden üblicherweise mehrere Schwellen verwendet. Typischerweise weisen die Detektorelemente Komparatoren auf, mit denen festgelegt werden kann, welche Mindestenergie eingehen muss, damit ein Signal gezählt wird. Das Prinzip der Verwendung von Schwellenwerten und Zählern kann sowohl zum Zählen von eintreffenden Photonen allgemein als auch zum Zählen von Koinzidenzen verwendet werden. Es kann vorgesehen sein, Koinzidenzeinheiten nur für eine, beispielsweise die niedrigste, oder für eine dedizierte eigene Schwelle vorzusehen. Es ist aber auch denkbar, Koinzidenzeinheiten für eine oder mehrere Schwellen oder gar für Kombinationen von unterschiedlichen Schwellen vorzusehen.

Der Röntgendetektor umfasst eine Vielzahl von Detektorelementen. Die Detektorelemente können Matrix-artig angeordnet sein. Insbesondere können mehrere Detektorelemente einem Teildetektor zugeordnet sein. Die Detektorelemente können gleichmäßig verteilt sein. Optional können die Detektorelemente gruppenweise angeordnet sein. Die Detektorelemente können auch mit anderen Bezeichnungen, wie Pixel, Pixelelemente, Bildpunktelemente etc. benannt werden. Generell ist der Begriff Detektorelement im Rahmen der Erfindung breit auszulegen. Abgesehen von den explizit genannten Merkmalen und der benötigten Funktionalität kann die Gestaltung der Detektorelemente relativ frei gewählt werden. So können beispielsweise einer, einige oder alle Komparatoren oder Zähler Teil eines Detektorelements sein oder separat ausgeführt sein.

Die Koinzidenzeinheit kann beispielsweise auch als Koinzidenzschaltung bezeichnet werden. Im Rahmen dieser Erfindung kann diese Koinzidenzeinheit zur Bestimmung zufälliger Koinzidenzen auch als Zufalls-Koinzidenzeinheit bezeichnet werden. Die Koinzidenzeinheit weist mehrere Signaleingänge auf und ist insbesondere dazu ausgestaltet, gleichzeitig eingehende Signale als Koinzidenzen zu erfassen und ein entsprechendes Zählsignal zum Zählen der Koinzidenzen zu erzeugen. Die Signaleingänge sind insbesondere als Eingang für die durch Röntgenstrahlung erzeugten elektrischen Signale vorgesehen. Die elektrischen Signale können auf Basis von Schwellwerten gefiltert werden. Beispielsweise kann es vorgesehen, sein, dass nur elektrische Signale mit einer definierten Mindeststärke zu dem ersten Signaleingang und/oder zu dem zweiten Signaleingang geleitet werden. Zu dem ersten Signaleingang werden Signale geleitet, die von einem ersten der Detektorelemente erfasst werden. Beispielsweise kann das erste der Detektorelemente dasjenige sein, für das die Rate der zufälligen Koinzidenzen erfasst wird. Alternativ kann das erste der Detektorelemente ein anderes als dasjenige sein, für das die Rate der zufälligen Koinzidenzen erfasst wird. Beispielsweise kann das erste der Detektorelemente ein Detektorelement sein, das in der Nähe des Detektorelements, für das die Rate der zufälligen Koinzidenzen erfasst wird, angeordnet ist. "In der Nähe" kann zum Beispiel benachbart, oder das übernächste Detektorelement bedeuten. Benachbart kann im Rahmen dieser Erfindung insbesondere bedeuten, dass das, ein Detektorelement in einer bestimmten Richtung das nächste Detektorelement zu dem Ausgangs-Detektorelement ist. Insbesondere befindet sich kein Detektorelement direkt zwischen zwei benachbarten Detektorelementen. In einer rechteckigen Matrix aus Detektorelementen werden auch Detektorelemente als benachbart bezeichnet, wenn sie schräg benachbart sind. Mit anderen Worten sind auch Detektorelemente benachbart, die nicht entlang der Rechteckseiten aufeinanderfolgen, sondern die schräg zu den Rechteckseiten aufeinanderfolgen. "Schräg" bedeutet im Rahmen dieser Erfindung insbesondere entlang einer Diagonalen der rechteckigen Matrix.

Gemäß einer Alternative kann es vorgesehen sein, dass die Signale des ersten Signaleingangs oder der weiteren Signaleingänge verzögert sind. Dies kann insbesondere durch eine Verzögerung in der elektrischen Schaltung realisiert werden. Vorzugsweise ist die Verzögerung größer als eine Signalverarbeitungszeit der Detektorelemente. Durch die Verzögerung kann ausgeschlossen werden, dass die Signale durch ein einziges Photon ausgelöst werden. Vorzugsweise ist die Verzögerung so gering gewählt, dass eine Änderung des Röntgenflusses in dem Zeitraum der Verzögerung statistisch vernachlässigbar ist. Da die zeitliche Abfolge von Signalen mehrerer Röntgenquanten unkorreliert ist, ändert sich die Zählrate der zufälligen Koinzidenzen nicht, wenn ein Signalpfad verzögert wird, solange diese Verzögerung kurz ist im Vergleich zu der Abtastung in einem Röntgengerät, beispielsweise in einem Computertomographie-Gerät. Da man somit von einer näherungsweise gleichbleibenden Statistik über den Zeitraum der Verzögerung ausgehen kann, kann ein gutes Maß für die zufällige Koinzidenz ermittelt werden, wobei gleichzeitig echte Koinzidenzen durch die Verzögerung im Wesentlichen ausgeschlossen werden können.

Gemäß einer weiteren Alternative stammen die Signale für den ersten Signaleingang und für den zumindest einen weiteren Signaleingang von Detektorelementen, die nicht direkt zueinander benachbart sind. Vorzugsweise können die Signale der weiteren Signaleingänge von Detektorelementen stammen, die mit Bezug zu dem ersten Detektorelement das jeweils übernächste Detektorelement sind. Mit anderen Worten kann es vorgesehen sein, dass zwischen dem ersten Detektorelement und den Detektorelementen für die weiteren Signaleingänge jeweils genau ein anderes Detektorelement angeordnet ist. Indem die Detektorelemente nicht direkt benachbart sind, kann weitgehend ausgeschlossen werden, dass dort erfasste Signale von dem gleichen Röntgenphoton stammen. Vorteilhafterweise bei dieser Variante ist, dass eine Verzögerungsschaltung nicht unbedingt benötigt wird. Auch müssen gegebenenfalls nicht die Verzögerungen mehrerer weiterer Signaleingänge koordiniert und angeglichen werden. Somit kann der Aufwand und/oder der benötigte Raum für die Schaltung verringert werden. Eine zufällige Koinzidenz kann somit über eine räumliche Trennung realisiert werden.

Optional vorzugsweise sind höchstens neun, besonders bevorzugt höchstens fünf Signaleingänge für die Koinzidenzeinheit vorgesehen. Beispielsweise können ganz besonders bevorzugt genau 5 oder genau 3 Signaleingänge vorgesehen sein. Von den Signaleingängen kann insbesondere ein Signaleingang für Signale von dem Detektorelement, für das eine zufällige Koinzidenz bestimmt werden soll, und die übrigen Signaleingänge für Signale von weiteren Detektorelementen vorgesehen sein. In manchen Ausführungsformen kann es besonders bevorzugt sein, maximal vier, ganz besonders bevorzugt genau zwei Signaleingänge für die Koinzidenzeinheit vorzusehen. Insbesondere kann es in diesen Ausführungsformen vorgesehen sein, dass die Signale für die Signaleingänge allesamt nicht von dem Detektorelement, für das eine zufällige Koinzidenz bestimmt werden soll, sondern von weiteren Detektorelementen kommen. Indem somit die Anzahl der Detektorelemente für das Abschätzen gering ist, kann das Auftreten der gezählten zufälligen Koinzidenzen geringgehalten werden. Somit steigt der Zähler weniger stark mit dem Röntgenfluss an und kann daher vorteilhafterwiese über einen ausgedehnteren Flussbereich Information liefern. Es hat sich gezeigt, dass auch mit zwei Signaleingängen in der Regel noch eine ausreichend gute Statistik zum Abschätzen der Rate der zufälligen Koinzidenzen erzeugt werden kann.

Optional können die beiden genannten Alternativen auch kombiniert werden.

Mit den Koinzidenzeinheiten können dann erfasste Koinzidenzen gezählt werden, um zumindest eine Zählrate von erfassten zufälligen Koinzidenzen zu bestimmen. Somit kann eine Zahl der zufälligen Koinzidenzen explizit gemessen werden.

Anhand der gezählten zufälligen Koinzidenzen kann die Rate von zufälligen Koinzidenzen abgeschätzt werden. Generell kann die Abschätzung genauer sein, wenn mehr elektrische Signale verwendet werden. Andererseits kann der Schaltungsaufwand geringer sein, wenn weniger elektrische Signale berücksichtigt werden. Die Rate der zufälligen Koinzidenzen kann je nach tatsächlichem Setup entsprechend skaliert werden.

Optional kann es vorgesehen sein, dass nicht alle registrierten elektrischen Signale, auch unabhängig von deren Stärke, für das Zählen von Koinzidenzen verwenden werden. Beispielsweise können nur exemplarisch Koinzidenzen für eine Auswahl an elektrischen Signalen ermittelt werden. Damit kann ein Schaltungsaufwand verringert werden. Ein Verwenden von weniger Signalen kann durch statistische Rechnungen bei der Abschätzung der Rate der zufälligen Koinzidenzen berücksichtigt werden.

Gemäß einer Ausführungsform werden die Signale für den zumindest einen weiteren Signaleingang jeweils in einem anderen der Detektorelemente, insbesondere jeweils einem zu dem ersten Detektorelement benachbarten Detektorelement, erfasst, wobei Signale für den zumindest einen weiteren Signaleingang oder die Signale für den ersten Signaleingang in der elektrischen Schaltung mit einem definierten Zeitintervall zeitlich versetzt werden, bevor sie zu der Koinzidenzeinheit weitergeleitet werden. Vorzugsweise übersteigt das Zeitintervall maximalen Laufzeitunterschiede der analogen und/oder der digitalen Signale, vorzugsweise von beiden, in der elektrischen Schaltung. Vorteilhafterweise kann eine echte Koinzidenz diesen Zähler nicht erhöhen. Beispielsweise kann das Zeitintervall in einem Bereich von 50ns bis 10 ps liegen, vorzugsweise weise 80ns bis 300ns, noch bevorzugter 100ns bis 200ns. Da Laufzeitunterschiede von Röntgendetektoren typischerweise unterhalb dieser Zeiten und typische Integrationszeiten von Computertomographiesystemen typischerweise deutlich höher liegen, sind diese Bereiche besonders günstig. Ein Bereich von über 100ns ist dabei besonders gut geeignet, um eine Abgrenzung zu typischen Laufzeiten und somit auch zu echten Koinzidenzen zu erlangen. Mit einer oberen Grenze von 300ns ist dabei besonders gut geeignet, um einen Einfluss eines sich zeitlich ändernden Röntgenflusses, z.B. durch einen sich weiterbewegenden Detektor vernachlässigbar gering zu halten. Ein Zeitintervall von maximal 200ns ist dagegen noch günstiger, da, durch diese Laufzeit, der Schaltungsaufwand weiter verringert werden kann und eine benötigte Leistung des Röntgendetektors verringert werden kann. Das Verwenden von benachbarten Detektorelementen kann dabei besonders vorteilhaft sein, weil somit ein entsprechend zu den echten Koinzidenzen sehr ähnliches oder sogar quasi identisches Setup erzeugt werden kann.

Gemäß einer Ausführungsform werden die Signale für den zumindest einen weiteren Signaleingang jeweils ebenfalls in dem ersten der Detektorelemente erfasst, wobei Signale für den zumindest einen weiteren Signaleingang oder die Signale für den ersten Signaleingang in der elektrischen Schaltung mit einem definierten Zeitintervall zeitlich versetzt werden, bevor sie zu der Koinzidenzeinheit weitergeleitet werden. Das Zeitintervall kann demjenigen der Ausführungsform mit verschiedenen Detektorelementen entsprechen. Bei dieser Ausführungsform kann ein Vorteil darin liegen, dass eine Verschaltung zwischen verschiedenen Detektorelementen verringert werden kann.

Gemäß einer Ausführungsform sind mehrere Koinzidenzeinheiten vorgesehen, von denen jeweils zumindest eine Koinzidenzeinheit einer Untergruppe von Detektorelementen zugeordnet ist, insbesondere einer Untergruppe aus örtlich beieinander angeordneten Detektorelementen, wobei das Verfahren für jede der mehreren Koinzidenzeinheiten angewandt wird, wobei mit jeder zumindest einen der mehreren Koinzidenzeinheiten jeweils die Rate von zufälligen Koinzidenzen für die Detektorelemente der zugehörigen Untergruppe abgeschätzt wird.

Gemäß einer Ausführungsform wird mit der zumindest einen Koinzidenzeinheit die Rate von zufälligen Koinzidenzen für ein abzuschätzendes Detektorelement abgeschätzt, dessen elektrisches Signal selbst nicht in die Koinzidenzeinheit geleitet wird, wobei die Signale für den ersten Signaleingang und die Signale für den zumindest einen weiteren Signaleingang von Detektorelementen stammen, die zu dem abzuschätzenden Detektorelement jeweils benachbart sind. Vorteilhafterweise können somit die zufälligen Koinzidenzen von Detektorelementen abgeleitet werden, die räumlich näher am betrachteten Detektorelement liegen, so dass räumliche Variationen der Zählraten über den Detektor weniger den Wert für die zufälligen Koinzidenzen beeinträchtigen kann. Vorzugsweise gibt es genau einen weiteren Signaleingang der Koinzidenzeinheit und die Signale für den ersten Signaleingang und für den einen weiteren Signaleingang stammen insbesondere von insgesamt zwei benachbarten Detektorelementen. Vorzugsweise sind die Detektorelemente in einer rechteckigen Matrix angeordnet und die insgesamt zwei benachbarten Detektorelemente für den ersten Signaleingang und für den einen weiteren Signaleingang sind schräg benachbart zu dem abzuschätzenden Detektorelement angeordnet.

Gemäß einer Ausführungsform ist zumindest eine Koinzidenzeinheit einer Untergruppe von Detektorelementen zugeordnet, insbesondere einer Untergruppe aus örtlich beieinander angeordneten Detektorelementen, wobei mit der zumindest einen Koinzidenzeinheit die Rate von zufälligen Koinzidenzen für die Detektorelemente der zugehörigen Untergruppe abgeschätzt wird. Durch das Verwenden einer Koinzidenzeinheit für eine gesamte Untergruppen kann ein Schaltungsaufwand reduziert werden. Es können aber auch mehrere Koinzidenzeinheiten, beispielsweise 2 bis 4 Koinzidenzeinheiten, für die Untergruppe vorgesehen sein. Insbesondere kann es so vorgesehen sein, die Schaltung zur Messung der zufälligen Koinzidenzen nur für einen Teil der Detektorelemente aufzubauen. Beispielsweise kann eine Untergruppe 2 bis 200, vorzugsweise 4 bis 100, besonders bevorzugt 10 bis 50, Detektorelemente umfassen. Die Untergruppe kann beispielsweise rechteckförmig angeordnet sein, wobei die Seitenlängen des Rechtecks durch die Anzahl der Detektorelemente in der Untergruppe definiert ist. Beispielsweise können die Seitenlängen des Rechteckes jeweils im Bereich von 2 bis 10 Detektorelementen liegen. Ein Bereich von 2 bis 10 Detektorelementen in jeder Richtung kann besonders günstig sein, indem sich in diesem Bereich die Zählraten typischerweise nicht zu stark unterscheiden und gleichzeitig eine gute Ersparnis an Schaltungsaufwand möglich ist. Vorzugsweise ist jeweils eine Koinzidenzeinheit pro 2 bis 36 Detektorelementen, besonders bevorzugt jeweils eine Koinzidenzeinheit pro 6 bis 24 Detektorelementen, vorgesehen.

Bevorzugt wird nur ein Paar von Detektorelementen für die Bestimmung der zufälligen Koinzidenzen innerhalb einer Untergruppe verwendet. Insbesondere ist vorzugsweise eine Koinzidenzeinheit pro Untergruppe vorgesehen, wobei die Koinzidenzeinheit genau zwei Signaleingänge für Signale von zwei Detektorelementen aufweist. Vorzugsweise können 4 bis 100, besonders bevorzugt 9 bis 36, Detektorelemente pro Untergruppe vorgesehen sein. Die Detektorelemente können in einer rechteckigen Matrix angeordnet sein. Die Matrix kann die Form N×M aufweisen, wobei N und M die Anzahl der Detektorelemente entlang der Seiten der rechteckigen Matrix sind. N und M können beispielsweise jeweils einen Wert von 2 bis 10, vorzugsweise von 3 bis 6, aufweisen. Beispielsweise können die Detektorelemente in einer rechteckigen 4×6-Matrix angeordnet sein. Es hat sich gezeigt, dass mit einer solchen Matrix in dieser Ausführungsform besonders gute Ergebnisse erzielt werden können, bei gleichzeitig effizienter Einsparung von Schaltungen. Vorzugsweise sind die Detektorelemente, die für die Signaleingänge der Detektoreinheit verwendet werden so in der Untergruppe angeordnet, dass jedes andere Detektorelement zumindest zu einem dieser beiden Detektorelemente zumindest ein übernächster Nachbar ist.

Gemäß einer Ausführungsform sind die Detektorelemente in Untergruppen von Detektorelementen aufgeteilt, wobei zwischen den Untergruppen Zwischenräume vorhanden sind, wobei zumindest eine Koinzidenzeinheit, insbesondere die Koinzidenzeinheit oder die mehreren Koinzidenzeinheiten, in den Zwischenräumen zwischen den Untergruppen angeordnet ist beziehungsweise angeordnet sind. Insbesondere kann die zumindest eine Koinzidenzeinheit in einem Bereich in dem Zwischenraum angeordnet sein, der im Schatten eines Anti-Streu-Grids liegt. Vorteilhafterweise kann in dieser Ausführungsform, die Schaltung für die Koinzidenzeinheit aus dem typischerweise begrenzten Raum direkt unter bzw. an den Detektorelementen in die Region zwischen den Detektorelementen verschoben sein. Der Raum direkt unter bzw. an den Detektorelementen ist typischerweise begrenzt, weil dort in der Regel bereits elektronische Schaltungen für die Verarbeitung der Signale der Detektorelemente angeordnet sind. Beispielsweise kann es vorgesehen sein, die Koinzidenzeinheiten nebeneinander angeordneter Untergruppen jeweils alternierend auf verschiedenen Seiten herauszuführen, insbesondere so, dass immer zwei Koinzidenzeinheiten gemeinsam in einem Zwischenraum sind. Dies kann, z.B. für die Weiterverarbeitung der Zählsignale der zufälligen Koinzidenzen, besonders vorteilhaft sein. Diese Variante kann mit anderen hierein beschriebenen Varianten, insbesondere Varianten betreffend Untergruppen, kombiniert werden. Insbesondere eine Variante mit einem Paar von Detektorelementen für die Bestimmung der zufälligen Koinzidenzen innerhalb einer Untergruppe kann vorteilhafterweise mit dieser Ausführungsform kombiniert werden.

Ein weiterer Aspekt der Erfindung ist ein Verfahren zum Abschätzen einer echten Koinzidenz zweier nach einem vorbestimmten Kriterium gleichzeitig erfasster Röntgensignale auf benachbarten Detektorelementen eines zählenden Röntgendetektors. Das Verfahren umfasst die folgenden Schritte:
- Durchführen des Verfahrens zum Abschätzen einer Rate von zufälligen Koinzidenzen wie hierin beschrieben; und
- Bestimmen der Koinzidenz zweier nach dem vorbestimmten Kriterium gleichzeitig erfasster Röntgensignale auf benachbarten Detektorelementen, um unkorrigierte Koinzidenzen zu ermitteln, insbesondere indem für jedes Detektorelement mit zumindest einem dazu benachbarten Detektorelement, optional mit jedem benachbarten Detektorelement, eine Anzahl an Koinzidenz-Zählsignalen gezählt wird;
- Korrigieren der unkorrigierten Koinzidenzen basierend auf der Rate von zufälligen Koinzidenzen, um die echte Koinzidenz abzuschätzen. Das vorbestimmte Kriterium kann beispielsweise einen Zeitraum umfassen, innerhalb dem Ereignisse als koinzident bzw. gleichzeitig gewertet werden. Die echte Koinzidenz kann insbesondere als Zählwert erfasst und/oder übertragen werden.

Alle Vorteile und Merkmale des Verfahrens zum Abschätzen einer Rate von zufälligen Koinzidenzen in einem zählenden Röntgendetektor können analog auf das Verfahren zum Abschätzen einer echten Koinzidenz übertragen werden und umgekehrt. Vorteilhafterweise kann durch die Ermittlung, insbesondere basierend auf einer expliziten Messung, der Beitrag von echten Koinzidenzen und von zufälligen in Koinzidenzzählern getrennt werden. Damit kann beispielsweise ein nutzbarer Messbereich für Koinzidenzzähler zu deutlich höheren Röntgenflüssen ausgedehnt werden und damit auch die Vorteile einer Koinzidenzschaltung. Einer möglichen Überkorrektur durch zu hoch abgeschätzte echte Koinzidenzen kann entgegengewirkt werden. Insbesondere kann dies ermöglicht werden, ohne auf Modellannahmen oder empirische Korrekturen angewiesen zu sein.

Das Bestimmen der Koinzidenz zweier nach dem vorbestimmten Kriterium gleichzeitig erfasster Röntgensignale auf benachbarten Detektorelementen kann beispielsweise gemäß einem im Stand der Technik bekannten Verfahren durchgeführt werden. Beispielsweise können mittels eines Koinzidenzzählers koinzident, d.h. heißt innerhalb einer bestimmten Toleranz gleichzeitig, erfasste elektrische Signale in benachbarten Detektorelementen registriert und gezählt werden. Zum Registrieren der Koinzidenzen kann beispielsweise eine Koinzidenzeinheit vorgesehen sein, die Fälle erfasst, in denen ein eingehendes elektrisches Signal einer jeweiligen Schwelle des Detektorelements in einem festgelegten Zeitrahmen gleichzeitig mit einem elektrischen Signal einer Schwelle von einem oder mehreren benachbarten Detektorelementen eingeht. Dazu können sowohl das elektrische Signal dieses Detektorelements als auch elektrische Signale der benachbarten Detektorelemente zu der Koinzidenzeinheit bzw. zu Signaleingängen der Koinzidenzeinheit geführt werden. Ein Koinzidenzzähler kann dann die Anzahl der Koinzidenzereignisse, bei denen mindestens ein benachbartes Detektorelement zeitgleich mit dem betrachteten Detektorelement eine Schwelle überschreitet, zählen.

Gemäß einer Ausführungsform wird mit der zumindest einen Koinzidenzeinheit die Rate von zufälligen Koinzidenzen für ein abzuschätzendes Detektorelement abgeschätzt, dessen elektrisches Signal selbst nicht in die Koinzidenzeinheit geleitet wird, wobei die Signale für den ersten Signaleingang und die Signale für den zumindest einen weiteren Signaleingang von Detektorelementen stammen, die zu dem abzuschätzenden Detektorelement jeweils benachbart sind. Vorzugsweise sind die Detektorelemente, von denen die Signale für die Signaleingänge stammen untereinander nicht benachbart. Vorzugsweise gibt es genau einen weiteren Signaleingang und die Signale für den ersten Signaleingang und für den einen weiteren Signaleingang stammen insbesondere von insgesamt zwei benachbarten Detektorelementen. Vorzugsweise sind die Detektorelemente in einer rechteckigen Matrix angeordnet und die insgesamt zwei benachbarten Detektorelemente für den ersten Signaleingang und für den einen weiteren Signaleingang sind schräg benachbart zu dem abzuschätzenden Detektorelement angeordnet. Vorzugsweise wird die unkorrigierte Koinzidenz basierend auf dem abzuschätzenden Detektorelement selbst und vier dazu benachbarten Detektorelementen durchgeführt, wobei die vier benachbarten Detektorelemente insbesondere vorzugsweise nicht die zwei benachbarten Detektorelemente für den ersten Signaleingang und für den einen weiteren Signaleingang sind. Insbesondere können die vier Detektorelementen, die für das Ermitteln der unkorrigierte Koinzidenz verwendet werden, entlang der Rechteckseiten der rechteckigen Matrix benachbart zu dem abzuschätzenden Detektorelement angeordnet.

Gemäß einer Ausführungsform ist sowohl zur Ermittlung der unkorrigierten Koinzidenzen als auch zur Abschätzung der zufälligen Koinzidenzen jeweils eine Koinzidenzeinheit vorgesehen, wobei für die beiden Koinzidenzeinheiten jeweils gleich viele Signaleingänge für elektrische Signale, deren Koinzidenzen gezählt werden, vorgesehen sind. Diese Ausführungsform kann eine besonders einfache Anpassung der unkorrigierten Koinzidenzen ermöglichen, insbesondere weil die Rate der zufälligen Koinzidenzen in der Regel nicht noch skaliert werden muss.

Gemäß einer Ausführungsform sind jeweils die gleichen Detektorelemente zur Bestimmung der unkorrigierten Koinzidenz als auch zur Abschätzung der zufälligen Koinzidenz vorgesehen, wobei insbesondere das Detektorelement, dessen echte Koinzidenz bestimmt werden soll, sowie dessen benachbarte Detektorelemente vorgesehen sind. Hiermit kann eine besonders genaue Abschätzung der zufälligen Koinzidenz möglich sein. Insbesondere werden die Signale des Detektorelements, dessen echte Koinzidenz bestimmt werden soll, oder die Signale der benachbarten Detektorelemente verzögert, bevor sie zu der Koinzidenzeinheit zur Abschätzung der zufälligen Koinzidenzen geleitet werden. Die zufällige Koinzidenz kann aufgrund des zeitlichen Versatzes mit einem definierten Zeitintervall bestimmt werden. Mit anderen Worten kann in dieser Ausführungsform die digitale Koinzidenzschaltung für jedes Detektorelement verdoppelt werden und für jedes Detektorelement können zwei Koinzidenzwerte gezählt werden. Dabei basiert der unkorrigierte Koinzidenzwert auf der Koinzidenz aus dem Signal eines Detektorelements und dem der dazu benachbarten Detektorelemente ohne Verzögerung. Dieser Wert repräsentiert insbesondere die Summe aus echten und zufälligen Koinzidenzen. Für den zweiten Signalpfad kann entweder das Signal des Detektorelements oder das Signal aller benachbarten Detektorelemente verzögert werden. Dieser Zählwert repräsentiert insbesondere die Anzahl der zufälligen Koinzidenzen.

Gemäß einer Ausführungsform sind zur Bestimmung der unkorrigierten Koinzidenz als auch zur Abschätzung der zufälligen Koinzidenz Signale für die Signaleingänge der jeweiligen Koinzidenzeinheit von dem Detektorelement, dessen echte Koinzidenz bestimmt werden soll, sowie von einem oder mehreren dessen benachbarter Detektorelemente vorgesehen, wobei nur eine Teilgruppe der benachbarten Detektorelemente zur Bestimmung der unkorrigierten Koinzidenz und/oder zur Abschätzung der zufälligen Koinzidenz vorgesehen ist. Gemäß einer alternativen Ausführungsform sind zur Bestimmung der zufälligen Koinzidenz Signale für die Signaleingänge der Koinzidenzeinheit zur Abschätzung der zufälligen Koinzidenzen von dem Detektorelement, dessen echte Koinzidenz bestimmt werden soll, sowie von dazu übernächsten Detektorelementen vorgesehen, wobei nur eine Teilgruppe der übernächsten Detektorelemente zur Abschätzung der zufälligen Koinzidenz und/oder nur eine Teilgruppe der benachbarten Detektorelemente zur Bestimmung der unkorrigierten Koinzidenz vorgesehen ist.

Indem nur eine Teilgruppe hinzugezogen wird, kann vorteilhafterweise der Schaltungsaufwand verringert werden. Die Teilgruppe kann optional aus nur einem Detektorelement bestehen. Zudem kann die so gezählte Anzahl an Koinzidenzen verringert werden. Dadurch können über einen ausgedehnteren Röntgenflussbereich Information zu zufälligen Koinzidenzen abgeschätzt werden. Bei der Anwendung eines solchen auf einer Teilgruppe basierenden Wertes zur Korrektur des Zählwertes zum Abschätzen der echten Koinzidenzen kann der Zählwert bzw. kann die Rate der zufälligen Koinzidenzen mit dem Verhältnis der Anzahl der benachbarten Detektorelemente, die für die Ermittlung der unkorrigierten Koinzidenzen verwendet werden, skaliert werden. Optional kann auch für die Ermittlung der unkorrigierten Koinzidenzen eine Teilgruppe, beispielsweise eine gleichgroße Teilgruppe oder eine Teilgruppe mit einer anderen Zahl an Detektorelementen, verwendet werden. Beispielsweise tragen schräg benachbarte Detektorelemente typischerweise weniger zu echten Koinzidenzen bei und es wird daher oftmals kein wesentlicher Fehler eingeführt, wenn auf diese verzichtet wird. Auch kann es sinnvoll sein, an den Rändern eines Detektors die Schaltung auf weniger Signaleingänge der jeweiligen Koinzidenzeinheit zu beschränken.

Gemäß einer Ausführungsform wird die echte Koinzidenz als Zählwert übertragen, wobei zusätzlich zu der echten Koinzidenz auch die zufällige Koinzidenz und/oder die unkorrigierte Koinzidenz jeweils als zusätzlicher Zählwert übertragen wird. Die zufällige Koinzidenz und/oder die unkorrigierte Koinzidenz ebenfalls zu übertragen kann es ermöglichen, diese bei einer weiteren Auswertung und oder zur nachträglichen Überprüfung der echten Koinzidenzen zu verwenden. Beispielsweise können die übertragenen Zählwerte der zufälligen Koinzidenzen in einem Rekonstruktionsrechner, in einem integrierten Schaltkreis, insbesondere FPGA (Field Programmable Gate Array), in einer internen Infrastruktur des Detektors und/oder in einer schaltungstechnischen äußeren Infrastruktur des Detektors mit anderen Zählwerten, z.B. den unkorrigierten Koinzidenzen, verrechnet werden. Vorteilhafterweise, kann somit beispielsweise nachträglich, z.B. anhand der tatsächlichen Zählwerte, entschieden werden, welche Korrekturen genutzt werden. Optional kann ein Beimischungsgrad der Korrektur dabei eine Funktion eines oder mehrerer weiterer Parameter sein. Weitere Parameter können beispielsweise die Zählrate, die Zahl der unkorrigierten oder echten Koinzidenzen, die Zahl der zufälligen Koinzidenzen und/oder andere, insbesondere Röntgenflussabhängige, Größen sein. Beispielsweise können sich bei sehr hohen Röntgenflüssen, lineare Pile-Up-Effekte häufen. Basierend darauf kann beispielsweise anhand des Beimischungsgrades ein fließender Übergang von der vollen Anwendung von Korrekturen zu einem Verwerfen der Zählwerte gesteuert werden. Beispielsweise kann der Beimischungsgrad ein Faktor von 1 sein und im Fall eines besonders großen Röntgenfluss ein Faktor von 0. In einer alternativen Ausführungsform kann es auch vorgesehen sein, dass nur die unkorrigierte Koinzidenz und die zufällige Koinzidenz übertragen wird. In diesem Fall kann es insbesondere vorgesehen sein, dass die echte Koinzidenz nachträglich bestimmt wird. In einer weiteren alternativen Ausführungsform kann es auch vorgesehen sein, dass nur die echte Koinzidenz übertragen wird, und insbesondere nicht die zufällige Koinzidenz und nicht die unkorrigierte Koinzidenz übertragen wird.

Gemäß einer Ausführungsform wird eine Zählrate der zufälligen Koinzidenzen verwendet, um eine Paralyse eines anderen Zählers von Signalen zu überwachen und gegebenenfalls zu korrigieren, insbesondere eine Paralyse eines anderen Zählers von Signalen, deren zufällige Koinzidenz mit dieser Zählrate abgeschätzt wird. Der andere Zähler kann beispielsweise ein Zähler zum Zählen eingehender Röntgenphotonen sein. Bei zu hohen Röntgenflüssen können zumindest manche Zähler paralysiert werden, indem sie nicht mehr in der Lage sind weitere Photonen zu erfassen. Dies kann sogar dazu führen, dass ein Zähler ab einer bestimmten Rate an eintreffenden Photonen wieder niedrigere Werte registriert. Dadurch können Mehrdeutigkeiten auftreten, weil nicht klar ist, ob der Zähler gerade einen tatsächlich niedrigen Röntgenfluss registriert oder ob der Zähler paralysiert ist. Der Zähler der zufälligen Koinzidenzen wird eher seltener paralysiert, weil Koinzidenzen von Ereignissen typischerweise seltener auftreten als einzelne Ereignisse. Paralysierbar können insbesondere Zähler mit einer niedrigen Energieschwelle sein, also Zähler die einen Großteil der eingehenden Photonen registrieren. Solche Zähler werden als paralysierbare Zähler bezeichnet. Vorteilhafterweise kann das Zählsignal des Zufalls-Koinzidenzzählers verwendet werden, um einen paralysierbare Zähler zu linearisieren, sodass insbesondere ein monoton steigendes, nichtparalysierbares Verhalten erreicht werden kann.

Gemäß einer Ausführungsform wird das Korrigieren der unkorrigierten Koinzidenzen basierend auf der Rate von zufälligen Koinzidenzen in dem Frontend des Röntgendetektors durchgeführt. Diese Ausführungsform kann relativ einfach zu implementieren sein, weil lediglich eine einfache Differenz der Zählwerte erzeugt werden muss. Eine Skalierung der Zählwerte kann in Form von einfachen Multiplikationen relativ einfach implementierbar sein. Wenn ein Röntgendetektor, z.B. in einem Computertomographiesystemen in Untergruppen unterteilt ist, gibt es typischerweise einige Kanten und Ecken, in denen die Anzahl der beitragenden Detektorelemente für sowohl die echten Koinzidenzen als auch für die zufälligen Koinzidenzen kleiner als für die Mehrzahl der übrigen Detektorelemente ist. Dieser Unterschied kann bei der Skalierung und gegebenenfalls auch durch eine Implementierung verschiedener Ausführungsformen berücksichtigt werden. Gerade für diesen Fall kann das Verrechnen bzw. Korrigieren in dem Frontend besonders vorteilhaft sein. Beispielsweise kann die genaue Verdrahtung, d. h. die jeweilige Anzahl der verwendeten Detektorelemente, an jeder Stelle bekannt sein und gezielt berücksichtigt werden.

Gemäß einer Ausführungsform wird zumindest einigen der von den Detektorelementen umgewandelten elektrischen Signale ein Pulsersignal beigemischt. Insbesondere wird auf maximal einen der Signaleingänge jeweils einer Koinzidenzeinheit ein Signal mit beigemischtem Pulsersignal gelegt. Das Pulsersignal kann beispielswiese für Kalibrationen, Totzeitmessungen und/oder zur Verhinderung einer Paralyse der Schaltung bei hohen Röntgenflüssen vorgesehen sein. Das Pulsersignal kann beispielsweise als Clock-Signal beigemischt werden. Durch regelmäßiges Einspeisen eines Clock-Signals kann ein Signal mitgezählt werden und somit eine Nichtparalysierbarkeit hergestellt werden. Pulsersignale sind typischerweise untereinander über große Detektorbereiche ganz oder teilweise korreliert. Indem auf maximal einen der Signaleingänge jeweils einer Koinzidenzeinheit ein Signal mit beigemischtem Pulsersignal gelegt wird, kann das Auftreten von systematischen Effekten, die den Zählwert durch künstlich erzeugte Koinzidenzen verfälschen können, verhindert werden.

Ein weiterer Aspekt der Erfindung ist ein Verfahren zum Aufnehmen eines Röntgenbilddatensatzes, insbesondere Computertomographiebilddatensatzes, eines Objekts mit einem Röntgensystem, insbesondere Computertomographiesystem, mit einem zählenden Röntgendetektor. Der Röntgendetektor umfasst eine Vielzahl von Detektorelementen. Das Verfahren umfasst die folgenden Schritte:
- Zählen zumindest einer Anzahl an Zählsignalen in Abhängigkeit der eintreffenden Röntgenstrahlung in jedem Detektorelement; und
- Durchführen eines Verfahrens zum Abschätzen einer echten Koinzidenz wie hierin beschrieben;
- Erzeugen eines Röntgenbilddatensatzes basierend auf der in jedem Detektorelement gezählten zumindest einen Anzahl an Zählsignalen und der abgeschätzten echten Koinzidenz.

Alle Vorteile und Merkmale des Verfahrens zum Abschätzen einer Rate von zufälligen Koinzidenzen in einem zählenden Röntgendetektor und des Verfahrens zum Abschätzen einer echten Koinzidenz können analog auf das Verfahren zur Aufnahme eines Röntgenbildes übertragen werden und umgekehrt. Beim Erzeugen des Röntgenbilddatensatzes können Bildwerte, insbesondere Voxelwerte bzw. Pixelwerte, basierend auf der zumindest einen Anzahl an Zählsignalen und der abgeschätzten echten Koinzidenz bereitgestellt werden. Die Anzahl an Zählsignalen kann insbesondere durch die echte Koinzidenz korrigiert bzw. angepasst werden. Insbesondere kann eine Bildrekonstruktion auf der angepassten zumindest einen Anzahl an Zählsignalen basieren. Beispielsweise kann eine Anzahl an Koinzidenz-Zählsignalen von der zumindest einen Anzahl an Zählsignalen subtrahiert werden. Beispielsweise kann eine gewichtete Subtraktion vorgesehen sein, insbesondere in dem vor der Subtraktion ein Beimischungsfaktor zu der Anzahl an Koinzidenz-Zählsignalen multipliziert wird. Das heißt, es kann lediglich ein Anteil oder ein Mehrfaches der zumindest einen Anzahl an Koinzidenz-Zählsignalen subtrahiert oder addiert werden. Für das Zählen zumindest einer Anzahl an Zählsignalen kann eine Schwelle verwendet werden, sodass Signale unterhalb dieser Schwelle nicht gezählt werden. Werden für ein Detektorelement mehrere Anzahlen an Zählsignalen gezählt, so kann für jeder der Anzahlen eine Schwelle verwendet werden, wobei insbesondere für die verschiedenen Anzahlen eines Detektorelements unterschiedliche Schwellen verwendet werden können. Die Koinzidenzen können für eine der Schwellen, die für die Anzahl an Zählsignalen verwendet werden, bestimmt werden. Beispielsweise kann die eine der Schwelle, die niedrigste der Schwellen sein. Das Verwenden nur einer Schwelle kann vorteilhafterweise einen Schaltungsaufwand verringern. Es ist aber auch denkbar, für die Koinzidenzeinheiten mehrere Schwellen oder für Kombinationen von unterschiedlichen Schwellen vorzusehen. Vorzugsweise werden zum Bestimmen der unkorrigierten Koinzidenz und der zufälligen Koinzidenz die gleiche Schwelle bzw. die gleichen Schwellen verwendet.

Gemäß einer Ausführungsform werden für das Zählen der Anzahl an Zählsignalen in jedem Detektorelement nur Röntgensignale erfasst, deren Energie eine erste Mindestschwelle überschreitet, wobei für die Bestimmung der zufälligen Koinzidenz nur Röntgensignale erfasst werden, deren Energie eine zweite Mindestschwelle überschreitet, wobei sich die erste Mindestschwelle und die zweite Mindestschwelle insbesondere unterscheidet. Es kann folglich eine dedizierte eigene Schwelle für die Koinzidenzmessung verwendet werden.

Ein weiterer Aspekt der Erfindung ist ein zählender Röntgendetektor, insbesondere für ein Computertomographiesystem, zur Aufnahme eines Röntgenbilddatensatzes eines von einer Röntgenstrahlung durchstrahlten Objekts, wobei der Röntgendetektor eine Vielzahl von Detektorelementen und zumindest eine elektrische Schaltung mit zumindest einer Koinzidenzeinheit umfasst, wobei der Röntgendetektor dazu konfiguriert ist, ein Verfahren wie hierin beschrieben auszuführen. Die zumindest eine elektrische Schaltung kann zumindest teilweise Teil eines Detektorelements sein. Beispielsweise kann jedes Detektorelement eine elektrische Schaltung aufweisen. Alle Vorteile und Merkmale des Verfahrens zum Abschätzen einer Rate von zufälligen Koinzidenzen in einem zählenden Röntgendetektor, des Verfahrens zum Abschätzen einer echten Koinzidenz und des Verfahrens zur Aufnahme eines Röntgenbildes können analog auf den zählenden Röntgendetektor übertragen werden und umgekehrt. Der Röntgendetektor kann einen Röntgenkonverter umfassen, in dem einfallende Röntgenstrahlen bewegliche Ladungsträger erzeugen. Die Detektorelemente können insbesondere dazu ausgestaltet sein, die von dem Röntgenkonverter erzeugten beweglichen Ladungsträger zu erfassen und als elektrisches Signal weiterzuarbeiten. Die Detektorelemente können jeweils zumindest einen Komparator umfassen. Der Komparator kann einen einstellbaren Signalschwellwert umfassen. Die Detektorelemente können Zähler zum Zählen von elektrischen Signalen umfassen, insbesondere wie hierin beschrieben.

Ein weiterer Aspekt der Erfindung ist ein medizinisches Bildgebungsgerät, insbesondere Computertomographiesystem, mit einem zählenden Röntgendetektor, insbesondere einem Röntgendetektor wie hierin beschrieben, und einem Steuermodul, wobei das medizinisches Bildgebungsgerät, insbesondere Computertomographiesystem, dazu konfiguriert ist, ein Verfahren wie hierin beschrieben auszuführen. Insbesondere kann das Steuermodul dazu ausgestaltet sein, das Ausführen des Verfahrens zu steuern. Das Steuermodul kann beispielsweise in Form eines Computers, eines Mikrocontrollers oder eines integrierten Schaltkreises umgesetzt sein oder jeweils ein Teil davon sein. Das Steuermodul kann Hardware-Elemente und/oder Software-Elemente aufweisen. Das Steuermodul kann optional ein Verbund von Computern oder eine Cloud sein, oder jeweils ein Teil davon sein. Alle Vorteile und Merkmale des Verfahrens zum Abschätzen einer Rate von zufälligen Koinzidenzen in einem zählenden Röntgendetektor, des Verfahrens zum Abschätzen einer echten Koinzidenz, des Verfahrens zur Aufnahme eines Röntgenbildes und des zählenden Röntgendetektors können analog auf das Computertomographiesystem übertragen werden und umgekehrt.

Alle hierin beschriebenen Ausführungsformen können miteinander kombiniert werden, soweit nicht explizit etwas anderes angegeben ist.

Im Folgenden werden Ausführungsformen mit Bezug auf die beigefügten Figuren beschrieben.
- Fig. 1: zeigt ein Flussdiagramm eines Verfahrens zum Abschätzen einer Rate von zufälligen Koinzidenzen in einem zählenden Röntgendetektor umfassend eine Vielzahl von Detektorelementen gemäß einer Ausführungsform der Erfindung,
- Fig. 2: zeigt ein Flussdiagramm eines Verfahrens zum Abschätzen einer echten Koinzidenz zweier nach einem vorbestimmten Kriterium gleichzeitig erfasster Röntgensignale auf benachbarten Detektorelementen eines zählenden Röntgendetektors gemäß einer Ausführungsform der Erfindung,
- Fig. 3: zeigt ein Flussdiagramm eines Verfahrens zur Aufnahme eines Röntgenbildes, insbesondere Computertomographiebildes, eines Objekts mit einem Röntgensystem, insbesondere Computertomographiesystem, gemäß einer Ausführungsform der Erfindung,
- Fig. 4: zeigt ein Schaltungsbeispiel eines Detektorelements gemäß einer Ausführungsform der Erfindung,
- Fig. 5: zeigt ein Schaltungsbeispiel eines Detektorelements gemäß einer weiteren Ausführungsform der Erfindung,
- Fig. 6: zeigt ein Schaltungsbeispiel eines Detektorelements gemäß einer weiteren Ausführungsform der Erfindung,
- Fig. 7: zeigt eine erfindungsgemäße Variante der in Figur 6 gezeigten Ausführungsform hinsichtlich von Möglichkeiten der Wahl der Detektorelemente, die zur Bestimmung der Koinzidenzen herangezogen werden,
- Fig. 8: zeigt eine erfindungsgemäße Variante der in Figur 6 gezeigten Ausführungsform hinsichtlich von Möglichkeiten der Wahl der Detektorelemente, die zur Bestimmung der Koinzidenzen herangezogen werden,
- Fig. 9: zeigt eine erfindungsgemäße Variante der in Figur 6 gezeigten Ausführungsform hinsichtlich von Möglichkeiten der Wahl der Detektorelemente, die zur Bestimmung der Koinzidenzen herangezogen werden,
- Fig. 10: zeigt eine weitere erfindungsgemäße Variante hinsichtlich einer Möglichkeit der Wahl der Detektorelemente, die zur Bestimmung der Koinzidenzen herangezogen werden,
- Fig. 11: zeigt eine weitere erfindungsgemäße Variante hinsichtlich einer Möglichkeit der Wahl der Detektorelemente, die zur Bestimmung der Koinzidenzen herangezogen werden,
- Fig. 12: zeigt ein Schaltungsbeispiel eines Detektorelements gemäß einer weiteren Ausführungsform der Erfindung,
- Fig. 13: zeigt verschiedene erfindungsgemäße Varianten der in Figur 12 gezeigten Ausführungsform hinsichtlich von Möglichkeiten der Wahl der Detektorelemente, die zur Bestimmung der Koinzidenzen herangezogen werden,
- Fig. 14: zeigt eine weitere erfindungsgemäße Variante hinsichtlich einer Möglichkeit der Wahl der Detektorelemente, die zur Bestimmung der Koinzidenzen herangezogen werden,
- Fig. 15: zeigt eine weitere erfindungsgemäße Variante hinsichtlich einer Möglichkeit der Wahl der Detektorelemente, die zur Bestimmung der Koinzidenzen herangezogen werden,
- Fig. 16: zeigt eine weitere erfindungsgemäße Variante hinsichtlich einer Möglichkeit der Wahl der Detektorelemente, die zur Bestimmung der Koinzidenzen herangezogen werden,
- Fig. 17: zeigt ein Variante der Platzierung der Schaltung zur Ermittlung der zufälligen Koinzidenz gemäß einer Ausführungsform der Erfindung, und
- Fig. 18: zeigt ein Computertomographiesystem gemäß einer Ausführungsform der Erfindung.

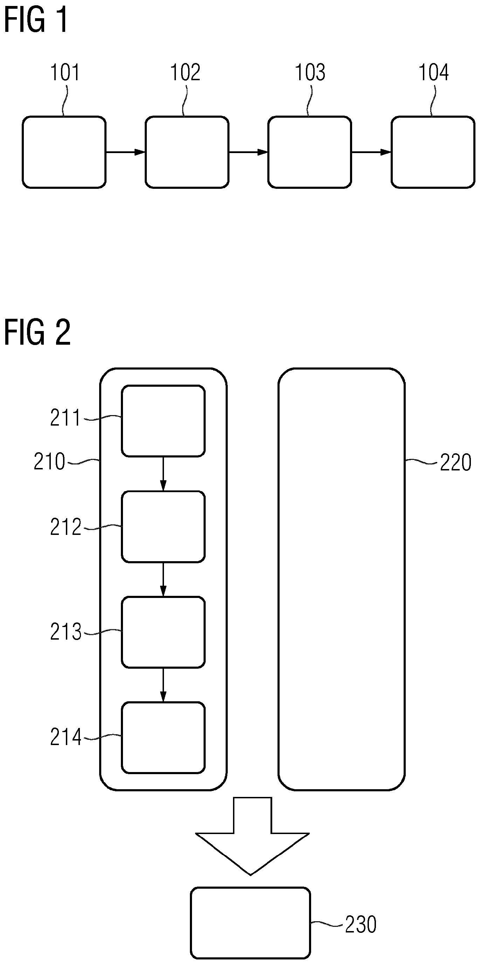

Figur 1 zeigt ein Flussdiagramm eines Verfahrens zum Abschätzen einer Rate von zufälligen Koinzidenzen in einem zählenden Röntgendetektor 31 umfassend eine Vielzahl von Detektorelementen gemäß einer Ausführungsform der Erfindung. In einem ersten Schritt 101 werden Röntgensignale durch den Röntgendetektor 31 erfasst und die Röntgensignale werden an den Detektorelementen in elektrische Signale umgewandelt. Das kann beispielsweise geschehen, indem Röntgenstrahlung in einem Röntgenkonverter in bewegliche Ladungsträger umgewandelt wird und dann durch eine angelegte Spannung in das Detektorelement an der jeweiligen Position eingespeist und dort verstärkt und mit einer Schwelle verglichen wird, sodass beim Überschreiten der Schwelle die elektrischen Signale ausgegeben werden. In einem weiteren Schritt 102 werden zumindest einige der elektrischen Signale zu Signaleingängen einer Koinzidenzeinheit weitergeleitet. Die Signaleingänge umfassen einen ersten Signaleingang und zumindest einen weiteren Signaleingang. Die Signale für den ersten Signaleingang werden in einem ersten der Detektorelemente erfasst. Die Signale für den zumindest einen weiteren Signaleingang werden jeweils in einem anderen nicht direkt zu dem ersten der Detektorelemente benachbarten Detektorelement erfasst. Indem die Signale für den zumindest einen weiteren Signaleingang in einem nicht direkt benachbarten Detektorelement erfasst werden, kann weitgehend sichergestellt werden, dass die Signale nicht von dem gleichen Röntgenphoton stammen, wie die Signale für den ersten Signaleingang. Zusätzlich oder alternativ werden die Signale für den zumindest einen weiteren Signaleingang oder die Signale für den ersten Signaleingang in einer elektrischen Schaltung mit einem definierten Zeitintervall zeitlich versetzt, bevor sie zu der Koinzidenzeinheit weitergeleitet werden. Durch den zeitlichen Versatz kann ebenfalls erreicht werden, dass die Signale des ersten Signaleingangs und des zumindest einen weiteren Signaleingangs nicht von dem gleichen Röntgenphoton stammen. In einem weiteren Schritt 103 werden Koinzidenzen der in die Koinzidenzeinheit weitergeleiteten Signale gezählt, um zumindest eine Zählrate von erfassten zufälligen Koinzidenzen zu bestimmen. Basierend auf der Zählrate wird in einem weiteren Schritt 104 eine Rate von zufälligen Koinzidenzen abgeschätzt. Die Rate von zufälligen Koinzidenzen kann direkt aus der Zählrate von erfassten zufälligen Koinzidenzen hervorgehen. Alternativ kann die Zählrate angepasst werden, insbesondere skaliert werden, um die Rate von zufälligen Koinzidenzen abzuschätzen.

Figur 2 zeigt ein Flussdiagramm eines Verfahrens zum Abschätzen einer echten Koinzidenz zweier nach einem vorbestimmten Kriterium gleichzeitig erfasster Röntgensignale auf benachbarten Detektorelementen eines zählenden Röntgendetektors 31 gemäß einer Ausführungsform der Erfindung. In einem übergeordneten Schritt 210 wird ein Verfahren zum Abschätzen einer Rate von zufälligen Koinzidenzen durchgeführt. Die Einzelschritte 211-214 dieses übergeordneten Schrittes 210 können beispielsweise den Schritten 101-104, wie sie in Bezug zu Figur 1 dargestellt sind, entsprechen. In einem weiteren übergeordneten Schritt 220 wird ein Verfahren zum Bestimmen der Koinzidenz zweier nach dem vorbestimmten Kriterium gleichzeitig erfasster Röntgensignale auf benachbarten Detektorelementen durchgeführt. Damit werden unkorrigierte Koinzidenzen ermittelt, insbesondere indem für zumindest einige Detektorelemente mit zumindest einem dazu benachbarten Detektorelement eine Anzahl an Koinzidenz-Zählsignalen gezählt wird. Die Anzahl an Koinzidenz-Zählsignalen kann beispielsweise in jedem Detektorelement oder in einem Teil der Detektorelemente gezählt werden. Das Zählen basiert auf dem in dem jeweiligen Detektorelement direkt eingegangenem Signal und auf einem koinzidierend auftretenden Signal mindestens eines benachbarten Detektorelements. Vorzugsweise werden diese beiden Schritte 210, 220 im Wesentlichen zur gleichen Zeit ausgeführt. In einem weiteren Schritt 230 werden die unkorrigierten Koinzidenzen basierend auf der Rate von zufälligen Koinzidenzen korrigiert, um die echte Koinzidenz abzuschätzen.

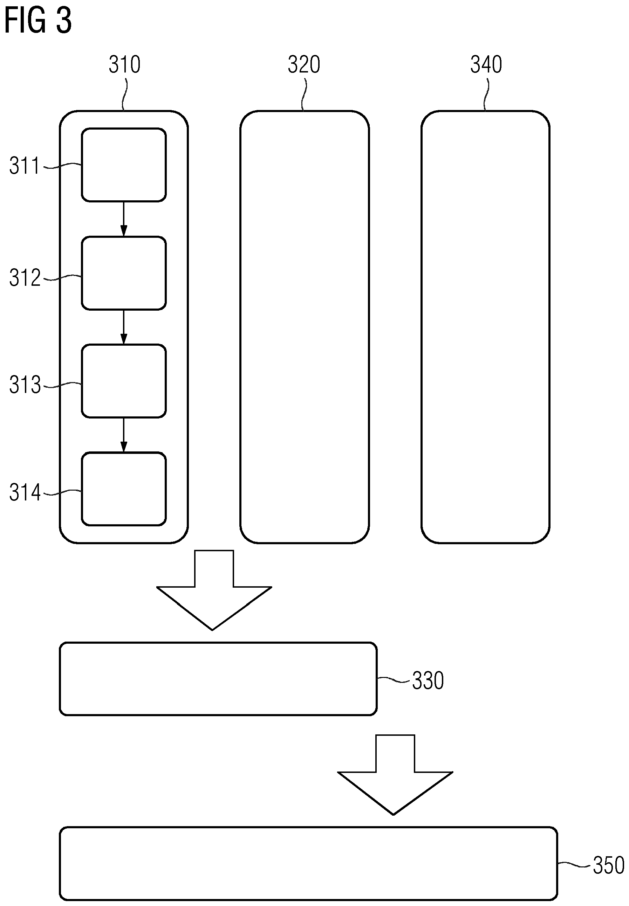

Figur 3 zeigt ein Flussdiagramm eines Verfahrens zur Aufnahme eines Röntgenbildes, insbesondere Computertomographiebildes, eines Objekts mit einem Röntgensystem, insbesondere Computertomographiesystem, gemäß einer Ausführungsform der Erfindung. Die Schritte 310-330 entsprechen den Schritten 210-230 des mit Bezug zu Figur 2 beschriebenen Verfahrens. In einem weiteren Schritt 340 wird in jedem Detektorelement zumindest einer Anzahl an Zählsignalen in Abhängigkeit der eintreffenden Röntgenstrahlung gezählt. Die Schritte 310, 320, die ein Verfahren zum Abschätzen einer Rate von zufälligen Koinzidenzen und ein Verfahren zum Bestimmen der Koinzidenz zweier nach gleichzeitig erfasster Röntgensignale betreffen können im Wesentlichen gleichzeitig mit dem weiteren Schritt 340, in dem in jedem Detektorelement zumindest einer Anzahl an Zählsignalen in Abhängigkeit der eintreffenden Röntgenstrahlung gezählt wird, durchgeführt werden. In einem weiteren Schritt 350 wird ein Röntgenbilddatensatz basierend auf der in jedem Detektorelement gezählten zumindest einen Anzahl an Zählsignalen und basierend auf der abgeschätzten echten Koinzidenz erzeugt.

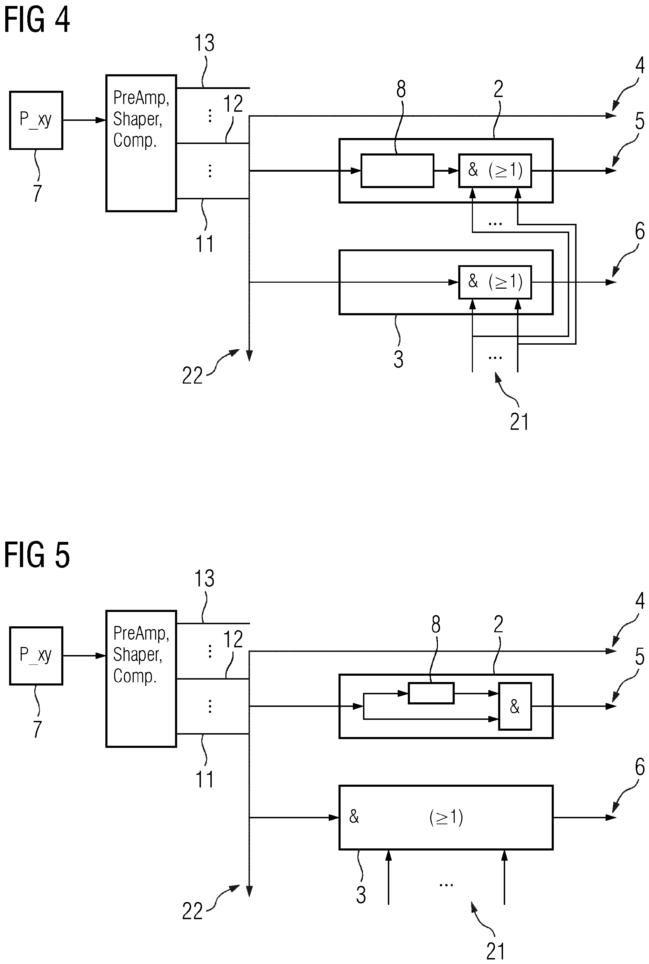

Figur 4 zeigt ein Schaltungsbeispiel eines Detektorelements gemäß einer Ausführungsform der Erfindung. Die Schaltung umfasst einen Eingang 7, in dem elektrische Signale, die aus eingehenden Röntgensignalen umgewandelt wurden, eingehen. Die elektrischen Signale werden verstärkt und mit mehreren Komparatoren 11, 12, 13, die N verschiedene Schwellwerte (Schwellwert 1, ... Schwellwert i, ... Schwellwert N) festlegen, gefiltert. Dadurch kann eingestellt werden, welche Mindestenergie registrierte Röntgensignale aufweisen müssen, um gezählt zu werden. Das Schaltungsbeispiel zeigt den Schaltungsbaum für einen der Schwellwerte 12. Elektrische Signale werden durch einen Zähler 4 gezählt. Dabei kann es vorkommen, dass nicht die gesamte Energie eines Röntgenquants in einem Detektorelement deponiert wird, sondern sich über zwei oder mehr - typischerweise benachbarte - Detektorelemente verteilt. Dies kann beispielsweise daran liegen, dass sich die im Detektor erzeugten Ladungswolken über mehr als einen Pixel ausdehnen. Um diesen Effekt zu berücksichtigen und zu korrigieren, ist eine Koinzidenzeinheit 3 vorgesehen. Die Koinzidenzeinheit 3 erfasst Fälle, in denen ein eingehendes elektrisches Signal der jeweiligen Schwelle 12 des Detektorelements in einem festgelegten Zeitrahmen gleichzeitig mit einem elektrischen Signal von einem oder mehreren benachbarten Detektorelementen eingeht. Dazu werden sowohl das elektrische Signal dieses Detektorelements als auch elektrische Signale 21 der benachbarten Detektorelemente zu der Koinzidenzeinheit 3 geführt. Ein Koinzidenzzähler 6 zählt dann die Anzahl der Koinzidenzereignisse, bei denen mindestens ein benachbartes Detektorelement zeitgleich mit dem betrachteten Detektorelement eine Schwelle überschreitet. Weil aber der Koinzidenzzähler 6 nicht nur im Falle von echten Koinzidenzen erhöht wird, sondern auch dann, wenn zeitgleich mit dem im betrachteten Detektorelement eintreffenden Röntgenquant zufällig ein zweites, unabhängiges Röntgenquant in einem der benachbarten Detektorelemente seine Energie deponiert, kann es zu einer Überkorrektur kommen, indem mehr echte Koinzidenzen gezählt werden, als eigentlich vorhanden sind. Gemäß dieser erfindungsgemäßen Ausführungsform ist eine weitere Koinzidenzeinheit 2 für zufällige Koinzidenzen vorgesehen (im Folgenden auch als Zufalls-Koinzidenzeinheit 2 bezeichnet). In dieser Ausführungsform werden mit der Zufalls-Koinzidenzeinheit 2 weitere Koinzidenzen, nämlich zwischen einem verzögerten Signal des Detektorelements und einem Signal 21 von benachbarten Detektorelementen gezählt. Entsprechend werden von diesem Detektorelement Signale 22 zu einer Zufalls-Koinzidenzeinheit 2 anderer Detektorelemente geleitet. Die Verzögerung wird in diesem Fall durch eine in die Zufalls-Koinzidenzeinheit 2 integrierte Verzögerung 8 erzielt, welche der eigentlichen Erfassung von Koinzidenzen vorgeschaltet ist und eingehende Signale mit einem definierten Zeitintervall zeitlich versetzt. Das Zeitintervall der Verzögerung wird dabei so gewählt, dass die Verzögerung die maximalen Laufzeitunterschiede sowohl der analogen als auch der digitalen Signale in der Schaltung übersteigt. Damit ist sichergestellt, das echte Koinzidenzen diesen Zufalls-Koinzidenzzähler 5 nie erhöhen können. Somit werden mit dem Zufalls-Koinzidenzzähler 5 nur zufällige Koinzidenzen gezählt. Da die zeitliche Abfolge von Signalen mehrerer Röntgenquanten unkorreliert ist, ändert sich die Zählrate der zufälligen Koinzidenzen nicht, wenn ein Signalpfad verzögert wird, solange diese Verzögerung kurz ist im Vergleich zu der Abtastung in einem CT-Gerät. Effektiv wird in dieser Ausführungsform die digitale Koinzidenzschaltung für jedes Detektorelement im Wesentlichen verdoppelt, sodass für jedes Detektorelement zwei Koinzidenzen gezählt werden. Die erste Koinzidenz wird mit der Koinzidenzeinheit 3 und dem Koinzidenzzähler 6 gezählt und erfasst Koinzidenzen aus dem Signal des Detektorelements und dem der Nachbarn, jeweils ohne Verzögerung. Dieser Wert repräsentiert die Summe aus echten und zufälligen Koinzidenzen. Für den zweiten Signalpfad wird das digitale Signal des Detektorelements verzögert und Koinzidenzen mit den benachbarten Detektorelementen werden mit der Zufallskoinzidenzeinheit 2 und dem Zufalls-Koinzidenzzähler 5 erfasst. Alternativ könnte beispielswiese auch das Signal 21 der benachbarten Detektorelemente verzögert werden. Dieser Zählwert des Zufalls-Koinzidenzzählers 5 repräsentiert die Anzahl der zufälligen Koinzidenzen. Im Folgenden kann man beispielsweise die Anzahl der zufälligen Koinzidenzen, erfasst durch den Zufalls-Koinzidenzzähler 5, von der Anzahl der gesamten Koinzidenzen, erfasst durch den Koinzidenzzähler 6, abziehen, so dass man die Anzahl der echten Koinzidenzen erhält. Wie hier dargestellt, können für die beiden Koinzidenzeinheiten 2, 3 jeweils gleich viele Signaleingänge für elektrische Signale, deren Koinzidenzen gezählt werden, vorgesehen sein.

Beispielsweise, um den Schaltungsaufwand zu minimieren, kann die Schaltung vereinfacht werden, indem der Koinzidenzbaum für die zufälligen Koinzidenzen nur teilweise aufgebaut wird. Demnach kann es optional vorgesehen sein, dass nicht alle benachbarten Detektorelemente für die Messung der zufälligen Koinzidenzen herangezogen werden, sondern nur eine Auswahl oder sogar nur ein einzelner. In dieser optionalen Varianten kann zur Korrektur des Zählwertes für die echten Koinzidenzen der Zählwert der Zufallskoinzidenzen mit dem Verhältnis der Anzahl der benachbarten Detektorelemente für die unkorrigierten Koinzidenzen der Koinzidenzeinheit 3 skaliert werden. Auch für die Koinzidenzeinheit 3 kann es optional vorgesehen sein, dass nicht Signale von allen benachbarten Detektorelementen herangezogen werden. Beispielsweise tragen die Nachbarn an den Ecken typischerweise wenig zu den echten Koinzidenzen bei und es kann daher zweckmäßig sein, auf diese zu verzichten. Auch an den Rändern eines Detektors, kann es sinnvoll sein, die Schaltung auf weniger Eingänge zu beschränken.

Bei der Beschreibung der folgenden Ausführungsbeispiele wird vor allem auf die Unterschiede der jeweiligen Ausführungsbeispiele eingegangen. Insbesondere werden Gemeinsamkeiten nicht für jede Figur erneut erläutert.

Figur 5 zeigt ein Schaltungsbeispiel eines Detektorelements gemäß einer weiteren Ausführungsform der Erfindung. In dieser Ausführungsform wird in der Zufalls-Koinzidenzeinheit 2 das elektrische Signal eines Detektorelements mit einer verzögerten Kopie dieses Signals selbst korreliert. Der Wert kann auch hier mit der Anzahl der Detektorelemente, deren Signal für die Koinzidenzeinheit verwendet werden, skaliert werden, um eine passende Korrektur der Zählwerte des Koinzidenzzählers 6 zu kontrollieren. Optional kann es vorgesehen sein, die Schaltung zur Messung der zufälligen Koinzidenzen nur für einen Teil der Detektorelemente aufzubauen, z. B. für jeweils ein oder zwei Detektorelemente in einer N×M Untergruppe von Detektorelementen, wobei N und M Werte vorzugsweise Werte zwischen 2 und 10 annehmen. Damit kann der Schaltungsaufwand vorteilhafterweise weiter reduziert werden.

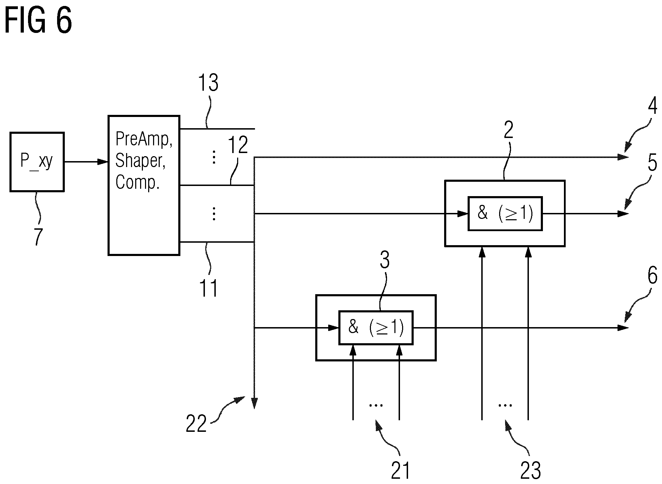

Figur 6 zeigt ein Schaltungsbeispiel eines Detektorelements gemäß einer weiteren Ausführungsform der Erfindung. In dieser Ausführungsform werden zufälligen Koinzidenzen über eine Korrelation von räumlich entfernten Detektorelementen erfasst. Dabei wird in der Zufalls-Koinzidenzeinheit 2 die Koinzidenz des Detektorelements mit den Signalen 23 der übernächsten oder noch weiter entfernten Detektorelemente gezählt. Da die Detektorelemente dann räumlich so deutlich getrennt sind, dass echte Koinzidenzen praktisch nicht auftreten können, werden hier nur zufällige Koinzidenzen gezählt. Vorteilhafterweise muss dafür keine zusätzliche Verzögerung 8 eingebaut werden, welche oftmals relativ aufwendig ist. Beispielsweise kann auch in dieser Ausführungsform die Koinzidenzschaltung für jedes Detektorelement verdoppelt werden, sodass gleich viele Signale zu der Koinzidenzeinheit 3 als auch zu der Zufalls-Koinzidenzeinheit 2 eingehen. In die Koinzidenzeinheit 3 gehen für die Messung der Koinzidenzen auch in dieser Ausführungsform Signale 21 von den direkt benachbarten Detektorelementen ein, womit alle Arten von Koinzidenzen (zufällige und echte Koinzidenzen) gemessen werden. Durch das Bestimmen der zufälligen Koinzidenzen durch den Zufalls-Koinzidenzzähler 5 können auch hier die echten Koinzidenzen herausgerechnet werden. In der in Figur 6 gezeigten Ausführungsform können optional für die beiden Koinzidenzeinheiten 2, 3 jeweils gleich viele Signaleingänge für elektrische Signale, deren Koinzidenzen gezählt werden, vorgesehen sein.

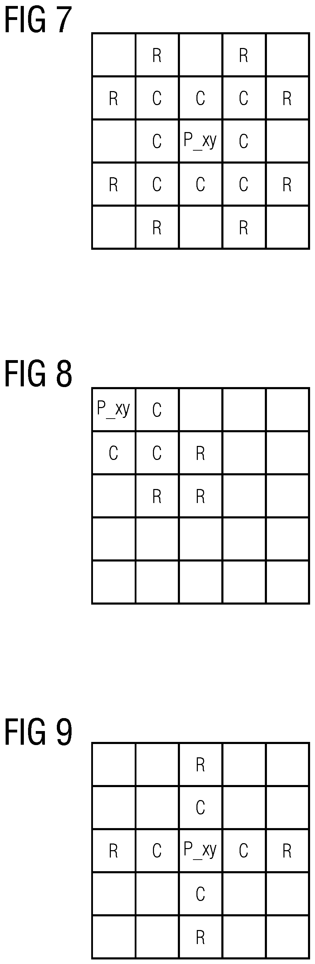

Die Figuren 7-9 zeigen verschiedene erfindungsgemäße Varianten der in Figur 6 gezeigten Ausführungsform hinsichtlich von Möglichkeiten der Wahl der Detektorelemente, die zur Bestimmung der Koinzidenzen herangezogen werden. Gezeigt ist jeweils eine Untergruppe von Detektorelementen, wobei die Detektorelemente durch die einzelnen Quadrate repräsentiert werden. P_xy steht dabei für das Detektorelement, dessen echte Koinzidenz bestimmt werden soll. Durch den Buchstaben C werden Detektorelemente gekennzeichnet, deren Signal 21 in die Koinzidenzeinheit 3 zum Bestimmen einer unkorrigierten Koinzidenz geleitet werden. Durch den Buchstaben R sind Detektorelemente gekennzeichnet, deren Signal 23 in die Zufalls-Koinzidenzeinheit 2 zum Bestimmen einer zufälligen Koinzidenz geleitet werden. Es sind natürlich auch andere Konfigurationen im Rahmen der Ausführungsform der Figur 6 denkbar. Es können auch verschiedene Varianten innerhalb eines Systems gemischt sein, z.B. für Kanten und Ecken eines Detektorabschnittes. In der Variante von Figur 7 werden alle benachbarten Detektorelemente C zur Bestimmung einer (unkorrigierten) Koinzidenz herangezogen. Zusätzlich wird die gleiche Anzahl Detektorelementen, allerdings in Gestalt von übernächsten Nachbarn R, zur Bestimmung der zufälligen Koinzidenz herangezogen. In diesem Beispiel werden alle übernächsten Nachbarn herangezogen, die in einem Rechteck übernächster Nachbarn nicht in den Ecken und nicht mittig auf den Seiten des Rechtecks angeordnet sind. In Figur 8 sitzt das Detektorelement P_xy, dessen echte Koinzidenz bestimmt werden soll, in einer Ecke der Untergruppe von Detektorelementen. Entsprechend weist es 3 Nachbarn C auf, welche zur Bestimmung der (unkorrigierten) Koinzidenz verwendet werden. Für die Bestimmung der zufälligen Koinzidenz werden entsprechend 3 übernächste Nachbarn R herangezogen. In diesem Beispiel werden die 3 übernächsten Nachbarn verwendet, die nicht am Rand der Untergruppe platziert sind. In der Ausführungsform gemäß Figur 9 werden jeweils 4 nächste Nachbarn C für die Bestimmung der (unkorrigierten) Koinzidenz herangezogen und 4 übernächste Nachbarn R für die Bestimmung der zufälligen Koinzidenz herangezogen. Dabei werden nur Nachbarn C bzw. übernächste Nachbarn R herangezogen, die in der rechteckigen Untergruppe von Detektorelementen auf der gleichen horizontalen oder vertikalen Linie liegen wie das Detektorelement P_xy, dessen Koinzidenz bestimmt wird.

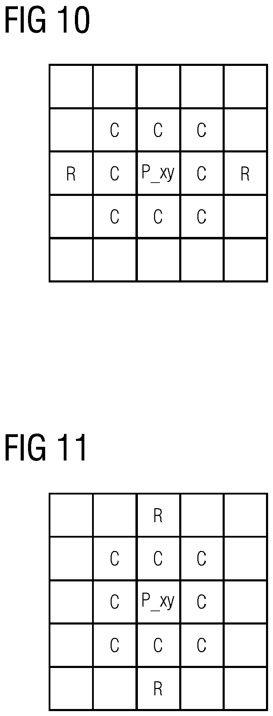

Die Figuren 10 und 11 zeigen weitere verschiedene erfindungsgemäße Varianten hinsichtlich von Möglichkeiten der Wahl der Detektorelemente, die zur Bestimmung der Koinzidenzen herangezogen werden. Die Benennung der Detektorelemente entspricht derjenigen in den Figuren 7 bis 9. Dabei wird für die Bestimmung der zufälligen Koinzidenzen eine kleinere Anzahl von Detektorelementen verwendet als für die Bestimmung der (unkorrigierten) Koinzidenzen. Insbesondere ist nur eine kleine Teilgruppe der übernächsten Detektorelemente zur Bestimmung der zufälligen Koinzidenz vorgesehen. Wie auch in der in Figur 7 gezeigten Variante, werden in den Ausführungsformen der Figuren 10 und 11 alle benachbarten Detektorelemente C zur Bestimmung der (unkorrigierten) Koinzidenzen herangezogen. Es werden jedoch nur jeweils 2 übernächste Nachbarn R zur Bestimmung der zufälligen Koinzidenzen herangezogen. In der in Figur 10 gezeigten Ausführungsform werden die beiden übernächsten Detektorelemente R in X-Richtung verwendet. In der in Figur 11 gezeigten Ausführungsform werden die beiden übernächsten Detektorelemente R in Y-Richtung verwendet. Vorzugsweise wird bei der Anwendung einer dieser beiden Varianten der Messwert der zufälligen Koinzidenzen mit einem Faktor K/2 skaliert, wobei K die Anzahl der Detektorelemente ist, die in der Koinzidenzeinheit 3 verwandt werden. In diesem Beispiel hätte K somit den Wert 8.

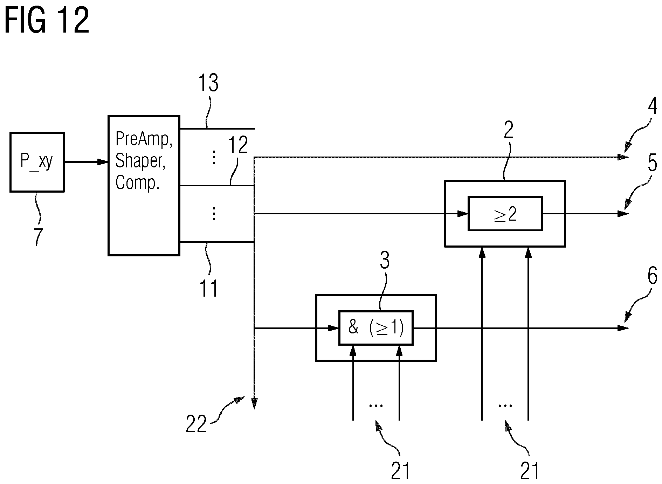

Figur 12 zeigt ein Schaltungsbeispiel eines Detektorelements gemäß einer weiteren Ausführungsform der Erfindung. In dieser Ausführungsform wird das elektrische Signal des Detektorelements, dessen Rate von zufälligen Koinzidenzen abgeschätzt wird, selbst nicht zur Bestimmung der zufälligen Koinzidenz verwendet. Die Signale 21, die zu der Zufalls-Koinzidenzeinheit 2 geleitet werden, stammen von Detektorelementen, die zu dem abzuschätzenden Detektorelement jeweils benachbart sind. Signale 21 von benachbarten Detektorelementen können sowohl nur in die Koinzidenzeinheit 3 oder nur in die Zufalls-Koinzidenzeinheit 2 gehen oder in beide. Detektorelemente, deren Signale in die Zufalls-Koinzidenzeinheit gehen, sind untereinander nicht benachbart.

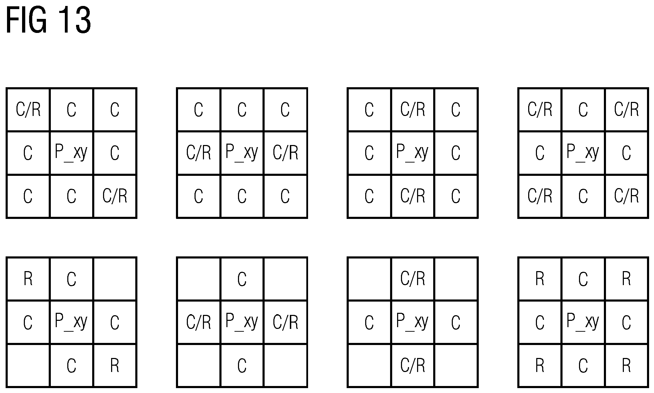

Figur 13 zeigt verschiedene erfindungsgemäße Varianten der in Figur 12 gezeigten Ausführungsform hinsichtlich von Möglichkeiten der Wahl der Detektorelemente, die zur Bestimmung der Koinzidenzen herangezogen werden. Die Benennung der Detektorelemente entspricht derjenigen in den Figuren 7 bis 11. In den Varianten werden entweder die Diagonale, linker und rechter Nachbar, oberer und unterer Nachbar, oder die 4 Ecken der gezeigten Untergruppe von Detektorelementen zur Messung der zufälligen Koinzidenzen herangezogen. In der oberen Reihe werden alle Nachbarn C, in der unteren nur die direkten Nachbarn C im Kreuz, d.h. nicht die schrägen Nachbarn, für die (unkorrigierte) Koinzidenzeinheit 3 genutzt. Prinzipiell sind auch weitere Kombinationen möglich. Es kann optional vorgesehen sein, mehrere Varianten in einem Detektor zu nutzen. Weil zu den zufälligen Koinzidenzen eine andere Anzahl an Detektorelementen beitragen als zu den unkorrigierten Koinzidenzen, können diese vorzugsweise, bevor sie zur Korrektur herangezogen werden, entsprechend skaliert werden. Die in den Figuren 12 und 13 gezeigten Varianten haben den Vorteil, dass die zufälligen Koinzidenzen von Detektorelementen abgeleitet werden, die räumlich näher an dem abzuschätzenden Detektorelement liegen, so dass räumliche Variationen der Zählraten über den Detektor weniger den Wert für die zufälligen Koinzidenzen beeinträchtigen können. Besonders vorteilhaft ist die links unten gezeigte Variante, da sie bei minimalem Verlust an wichtigen Informationen den kleinsten Schaltungsaufwand darstellt. In dieser Variante werden zwei übernächste Nachbarn R, die auf einer Diagonalen liegen zur Messung der zufälligen Koinzidenzen herangezogen und es werden nur die nächsten Nachbarn C die nicht die schrägen Nachbarn sind, für die (unkorrigierte) Koinzidenzeinheit 3 genutzt.

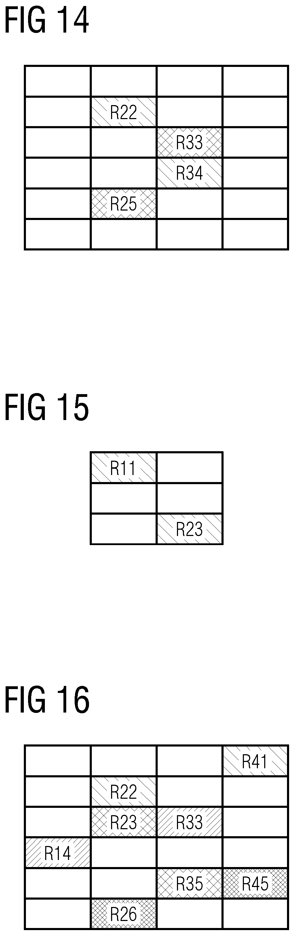

Die Figuren 14 bis 16 zeigen weitere verschiedene erfindungsgemäße Varianten hinsichtlich von Möglichkeiten der Wahl der Detektorelemente, die zur Bestimmung der Koinzidenzen herangezogen werden. Gezeigt sind jeweils M×N-Untergruppe von Detektorelementen. Dabei wird die Schaltung zur Messung der zufälligen Koinzidenzen nicht für jedes Detektorelement einzeln aufbaut, sondern für eine M×N-Untergruppe wird nur ein Detektorelement oder werden einige wenige Detektorelemente zur Messung der zufälligen Koinzidenzen herangezogen. Dadurch kann der Schaltungsaufwand noch weiter reduziert werden. In der in Figur 14 gezeigten Variante werden für eine Untergruppe von 4×6 Detektorelementen zwei getrennte Zufalls-Koinzidenzzähler 5 für zufällige Koinzidenzen aufgebaut, die repräsentativ für alle 24 Detektorelemente sind. Die Zufalls-Koinzidenzeinheiten 2 der Zufalls-Koinzidenzzähler 5 erhalten jeweils Signale von zwei Detektorelementen (R22 und R34 bzw. R33 und R25). Die Summe der beiden Zählwerte der zufälligen Koinzidenzen kann mit dem Faktor K/2 als Korrekturwert skaliert werden, wobei K die Anzahl der benachbarten Detektorelemente ist, die in die Koinzidenzlogik für die (unkorrigierten) Koinzidenzen eingeht. In der in Figur 15 gezeigten Variante ist in einer 2×3-Untergruppe ein Zufalls-Koinzidenzzähler 5 für alle Detektorelemente der Untergruppe vorgesehen, wobei die Zufalls-Koinzidenzeinheiten 2 der Zufalls-Koinzidenzzähler 5 jeweils Signale von zwei Detektorelementen (R11 und R23) erhalten. In der in Figur 16 gezeigten Variante sind in einer 4×6-Untergruppe vier Zufalls-Koinzidenzzähler 5 für alle Detektorelemente der Untergruppe vorgesehen, wobei die Zufalls-Koinzidenzeinheiten 2 der Zufalls-Koinzidenzzähler 5 jeweils Signale von zwei Detektorelementen (R22 und R41 bzw. R14 und R33 bzw. R23 und R35 bzw. R45 und R26) erhalten.

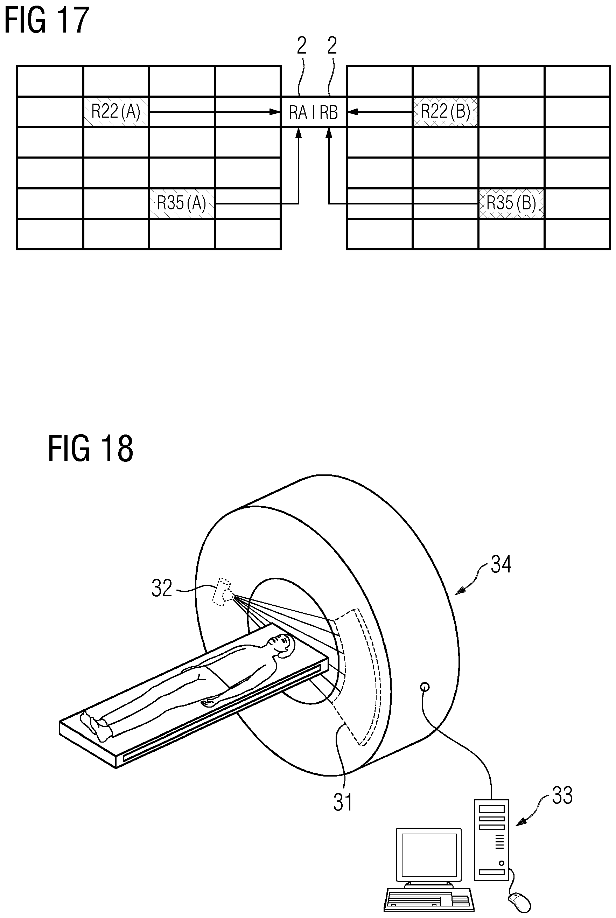

Figur 17 zeigt ein Variante der Platzierung der Schaltung zur Ermittlung der zufälligen Koinzidenz gemäß einer Ausführungsform der Erfindung. Die Detektorelemente, hier repräsentiert durch Rechtecke, sind in Untergruppen von Detektorelementen aufgeteilt, von denen hier zwei zu sehen sind. In dieser Ausführungsform werden in je zwei benachbarten Untergruppen jeweils zwei weit entfernte zentrale Detektorelemente für die Ermittlung der zufälligen Koinzidenz herangezogen. Zwischen den Untergruppen sind Zwischenräume. Die Zufalls-Koinzidenzeinheiten 2 der beiden gezeigten Untergruppen sind in dem Zwischenraum zwischen den Untergruppen angeordnet. Entsprechend können weitere Zufalls-Koinzidenzeinheiten 2 weiterer Untergruppen angeordnet sein. Wenn in einer Schaltung, z.B. einem ASIC, mehrere 4×6 Untergruppen aufgebaut sind, kann es vorgesehen sein, die Zufallskoinzidenzeinheiten abwechselnd links und rechts herauszuführen. Die Schaltung ist dabei aus dem begrenzten Raum unter den Detektorelementen in die Region zwischen den Untergruppen von Detektorelementen verschoben, die ansonsten eventuell ungenutzt wäre. Vorteilhafterweise wird die Zufallskoinzidenzschaltung somit außerhalb der eigentlichen Matrix von Detektorelementen platziert. Hierdurch können die Detektorelemente gleichartig ausgeführt werden und der vollständige Raum bei den Detektorelementen steht für die Schaltungen zur Verfügung, die für alle Detektorelemente vorhanden sein müssen. Die Aufteilung und Anordnung der Detektorelemente kann auch anders als gezeigt, beispielsweise wie in den Figuren 14 bis 16, sein.

Figur 18 zeigt ein Computertomographiesystem gemäß einer Ausführungsform der Erfindung. Das Computertomographiesystem umfasst ein Steuermodul 33, eine Röntgenquelle 32 und einen zählenden Röntgendetektor 31 mit einer Vielzahl von Detektorelementen und einer elektrischen Schaltung wie hierin beschrieben, beispielsweise wie in den Figuren 4 bis 17 beschrieben. Das Computertomographiesystem ist dazu konfiguriert, ein Verfahren wie hierin beschrieben, beispielsweise mit Bezug zu einer der Figuren 1 bis 3, auszuführen. Röntgenquelle 32 und Röntgendetektor 31 sind rotierbar in einer Gantry 34 angeordnet.

## Patentansprüche

1. Verfahren zum Abschätzen einer Rate von zufälligen Koinzidenzen in einem zählenden Röntgendetektor (31), wobei der Röntgendetektor (31) eine Vielzahl von Detektorelementen umfasst, wobei das Verfahren die folgenden Schritte umfasst:
(a) Erfassen von Röntgensignalen durch den Röntgendetektor (31) und Umwandeln der Röntgensignale in elektrische Signale an den Detektorelementen;
(b) Weiterleiten von zumindest einigen der elektrischen Signale zu Signaleingängen einer Koinzidenzeinheit (2), wobei die Signaleingänge einen ersten Signaleingang und zumindest einen weiteren Signaleingang umfassen,
- wobei die Signale für den ersten Signaleingang in einem ersten der Detektorelemente erfasst werden,
- wobei die Signale für den zumindest einen weiteren Signaleingang jeweils in einem anderen nicht direkt zu dem ersten der Detektorelemente benachbarten Detektorelement erfasst werden
und/oder
wobei die Signale für den zumindest einen weiteren Signaleingang oder die Signale für den ersten Signaleingang in einer elektrischen Schaltung mit einem definierten Zeitintervall zeitlich versetzt werden, bevor sie zu der Koinzidenzeinheit (2) weitergeleitet werden;
(c) Zählen von Koinzidenzen der in die Koinzidenzeinheit (2) weitergeleiteten Signale, um zumindest eine Zählrate von erfassten zufälligen Koinzidenzen zu bestimmen;
(d) Abschätzen einer Rate von zufälligen Koinzidenzen anhand der zumindest einen bestimmten Zählrate.

2. Verfahren gemäß Anspruch 1,
wobei die Signale für den zumindest einen weiteren Signaleingang jeweils in einem anderen der Detektorelemente, insbesondere jeweils einem zu dem ersten Detektorelement benachbarten Detektorelement, erfasst werden,
wobei die Signale für den zumindest einen weiteren Signaleingang oder die Signale für den ersten Signaleingang in der elektrischen Schaltung mit einem definierten Zeitintervall zeitlich versetzt werden, bevor sie zu der Koinzidenzeinheit (2) weitergeleitet werden.

3. Verfahren gemäß Anspruch 1,
wobei die Signale für den zumindest einen weiteren Signaleingang jeweils ebenfalls in dem ersten der Detektorelemente erfasst werden,
wobei die Signale für den zumindest einen weiteren Signaleingang oder die Signale für den ersten Signaleingang in der elektrischen Schaltung mit einem definierten Zeitintervall zeitlich versetzt werden, bevor sie zu der Koinzidenzeinheit (2) weitergeleitet werden.

4. Verfahren gemäß einem der vorhergehenden Ansprüche,
wobei mehrere Koinzidenzeinheiten (2) vorgesehen sind, von denen jeweils zumindest eine Koinzidenzeinheit (2) einer Untergruppe von Detektorelementen zugeordnet ist, insbesondere einer Untergruppe aus örtlich beieinander angeordneten Detektorelementen,
wobei das Verfahren für jede der mehreren Koinzidenzeinheiten (2) angewandt wird,
wobei mit jeder zumindest einen der mehreren Koinzidenzeinheiten (2) jeweils die Rate von zufälligen Koinzidenzen für die Detektorelemente der zugehörigen Untergruppe abgeschätzt wird.

5. Verfahren gemäß einem der vorhergehenden Ansprüche,
wobei mit der zumindest einen Koinzidenzeinheit (2) die Rate von zufälligen Koinzidenzen für ein abzuschätzendes Detektorelement abgeschätzt wird, dessen elektrisches Signal selbst nicht in die Koinzidenzeinheit (2) geleitet wird,
wobei die Signale für den ersten Signaleingang und die Signale für den zumindest einen weiteren Signaleingang von Detektorelementen stammen, die zu dem abzuschätzenden Detektorelement jeweils benachbart sind.

6. Verfahren gemäß einem der vorhergehenden Ansprüche,
wobei die Detektorelemente in Untergruppen von Detektorelementen aufgeteilt sind, wobei zwischen den Untergruppen Zwischenräume vorhanden sind,
wobei die Koinzidenzeinheit (2) oder die mehreren Koinzidenzeinheiten (2) in den Zwischenräumen zwischen den Untergruppen angeordnet ist beziehungsweise angeordnet sind.

7. Verfahren zum Abschätzen einer echten Koinzidenz zweier nach einem vorbestimmten Kriterium gleichzeitig erfasster Röntgensignale auf benachbarten Detektorelementen eines zählenden Röntgendetektors (31),
wobei der Röntgendetektor (31) eine Vielzahl von Detektorelementen umfasst, wobei das Verfahren die folgenden Schritte umfasst:
- Durchführen des Verfahrens zum Abschätzen einer Rate von zufälligen Koinzidenzen gemäß einem der vorhergehenden Ansprüche;
- Bestimmen der Koinzidenz zweier nach dem vorbestimmten Kriterium gleichzeitig erfasster Röntgensignale auf benachbarten Detektorelementen, um unkorrigierte Koinzidenzen zu ermitteln, insbesondere indem für jedes Detektorelement mit zumindest einem dazu benachbarten Detektorelement, optional mit jedem benachbarten Detektorelement, eine Anzahl an Koinzidenz-Zählsignalen gezählt wird;
- Korrigieren der unkorrigierten Koinzidenzen basierend auf der Rate von zufälligen Koinzidenzen, um die echte Koinzidenz abzuschätzen.

8. Verfahren gemäß Anspruch 7,
wobei sowohl zur Ermittlung der unkorrigierten Koinzidenzen als auch zur Abschätzung der zufälligen Koinzidenzen jeweils eine Koinzidenzeinheit (2, 3) vorgesehen ist, wobei für die beiden Koinzidenzeinheiten (2, 3) jeweils gleich viele Signaleingänge für elektrische Signale, deren Koinzidenzen gezählt werden, vorgesehen sind.

9. Verfahren gemäß Anspruch 8,
wobei zur Bestimmung der unkorrigierten Koinzidenz als auch zur Abschätzung der zufälligen Koinzidenz Signale für die Signaleingänge der jeweiligen Koinzidenzeinheit (2, 3) von dem Detektorelement, dessen echte Koinzidenz bestimmt werden soll, sowie von einem oder mehreren dessen benachbarter Detektorelemente vorgesehen sind,
wobei nur eine Teilgruppe der benachbarten Detektorelemente zur Bestimmung der unkorrigierten Koinzidenz und/oder zur Abschätzung der zufälligen Koinzidenz vorgesehen ist.

10. Verfahren gemäß Anspruch 8,
wobei zur Bestimmung der zufälligen Koinzidenz Signale für die Signaleingänge der Koinzidenzeinheit (2) zur Abschätzung der zufälligen Koinzidenzen von dem Detektorelement, dessen echte Koinzidenz bestimmt werden soll, sowie von dazu übernächsten Detektorelementen vorgesehen sind,
wobei nur eine Teilgruppe der benachbarten Detektorelemente zur Bestimmung der unkorrigierten Koinzidenz und/oder nur eine Teilgruppe der übernächsten Detektorelemente zur Abschätzung der zufälligen Koinzidenz vorgesehen ist.

11. Verfahren gemäß einem der Ansprüche 7 bis 10,
wobei die echte Koinzidenz als Zählwert übertragen wird,
wobei zusätzlich zu der echten Koinzidenz auch die zufällige Koinzidenz und/oder die unkorrigierte Koinzidenz jeweils als zusätzlicher Zählwert übertragen wird.

12. Verfahren gemäß einem der Ansprüche 7 bis 11,
wobei eine Zählrate der zufälligen Koinzidenzen verwendet wird, um eine Paralyse eines anderen Zählers von Signalen zu überwachen und gegebenenfalls zu korrigieren, insbesondere eine Paralyse eines anderen Zählers von Signalen, deren zufällige Koinzidenz mit dieser Zählrate abgeschätzt wird.

13. Verfahren zum Aufnehmen eines Röntgenbilddatensatzes, insbesondere Computertomographiebilddatensatzes, eines Objekts mit einem Röntgensystem, insbesondere Computertomographiesystem, mit einem zählenden Röntgendetektor (31),
wobei der Röntgendetektor (31) eine Vielzahl von Detektorelementen umfasst,
wobei das Verfahren die folgenden Schritte umfasst:
- Zählen zumindest einer Anzahl an Zählsignalen in Abhängigkeit der eintreffenden Röntgenstrahlung in jedem Detektorelement;
- Durchführen eines Verfahrens gemäß einem der Ansprüche 7 bis 12;
- Erzeugen eines Röntgenbilddatensatzes basierend auf der in jedem Detektorelement gezählten zumindest einen Anzahl an Zählsignalen und der abgeschätzten echten Koinzidenz.

14. Zählender Röntgendetektor (31), insbesondere für ein Computertomographiesystem, zur Aufnahme eines Röntgenbilddatensatzes eines von einer Röntgenstrahlung durchstrahlten Objekts,
wobei der Röntgendetektor (31) eine Vielzahl von Detektorelementen und zumindest eine elektrische Schaltung mit zumindest einer Koinzidenzeinheit (2) umfasst,
wobei der Röntgendetektor (31) dazu konfiguriert ist, ein Verfahren gemäß einem der vorhergehenden Ansprüche auszuführen.

15. Medizinisches Bildgebungsgerät, insbesondere Computertomographiesystem, mit einem zählenden Röntgendetektor (31), insbesondere einem Röntgendetektor (31) gemäß Anspruch 14, und einem Steuermodul (33), wobei das medizinische Bildgebungsgerät dazu konfiguriert ist, ein Verfahren gemäß einem der Ansprüche 1 bis 13 auszuführen.
